# EUROPEAN PATENT SPECIFICATION

(11) **EP 3 382 029 B1**
(45) Date of publication and mention of the grant of the patent: **08.12.2021**
(21) Application number: 18164547.4
(22) Date of filing: 28.03.2018
(51) Int. Cl.: C12N 15/90

(54) **RECOMBINANT MAMMALIAN CELLS AND METHOD FOR PRODUCING SUBSTANCE OF INTEREST**
REKOMBINANTE SÄUGETIERZELLEN UND VERFAHREN ZUR HERSTELLUNG EINER INTERESSIERENDEN SUBSTANZ
CELLULES RECOMBINANTES DE MAMMIFÈRES ET PROCÉDÉ DE PRODUCTION D'UNE SUBSTANCE D'INTÉRÊT

(30) Priority: 31.03.2017 JP 2017071936
(43) Date of publication of application: 03.10.2018
(73) Proprietor: Toto Ltd., Fukuoka 802-8601 (JP); Kyushu University National University Corporation, Fukuoka 812-8581 (JP)
(72) Inventor: YAMANA, Yoshimasa, Kitakyushu-shi, Fukuoka 8028601 (JP); KONDO, Masako, Kitakyushu-shi, Fukuoka 8028601 (JP); KAMIHIRA, Masamichi, Fukuoka-shi, Fukuoka 8128581 (JP)
(74) Representative: ABG Intellectual Property Law, S.L.

(56) References cited:
- YOSHINORI KAWABE ET AL: "Repeated integration of antibody genes into a pre-selected chromosomal locus of CHO cells using an accumulative site-specific gene integration system", CYTOTECHNOLOGY., vol. 64, no. 3, 25 September 2011 (2011-09-25), pages 267-279, XP055333210, NL ISSN: 0920-9069, DOI: 10.1007/s10616-011-9397-y
- KAMEYAMA YUJIRO ET AL: "An accumulative site-specific gene integration system using Cre recombinase-mediated cassette exchange", BIOTECHNOLOGY AND BIOENGINEERING, WILEY, US, vol. 105, no. 6, 15 April 2010 (2010-04-15), pages 1106-1114, XP002626345, ISSN: 0006-3592, DOI: 10.1002/BIT.22619 [retrieved on 2009-12-07]
- HIROKAZU OBAYASHI ET AL: "Accumulative gene integration into a pre-determined site using Cre/", JOURNAL OF BIOSCIENCE AND BIOENGINEERING, ELSEVIER, AMSTERDAM, NL, vol. 113, no. 3, 31 October 2011 (2011-10-31), pages 381-388, XP028463093, ISSN: 1389-1723, DOI: 10.1016/J.JBIOSC.2011.10.027 [retrieved on 2011-11-07]
- INAO TAKANORI ET AL: "Improved transgene integration into the Chinese hamster ovary cell genome using the Cre-loxP system", JOURNAL OF BIOSCIENCE AND BIOENGINEERING, vol. 120, no. 1, July 2015 (2015-07), pages 99-106, XP002783294,
- KAWABE YOSHINORI ET AL: "Improved recombinant antibody production by CHO cells using a production enhancer DNA element with repeated transgene integration at a predetermined chromosomal site", JOURNAL OF BIOSCIENCE AND BIOENGINEERING, ELSEVIER, AMSTERDAM, NL, vol. 123, no. 3, 14 November 2016 (2016-11-14), pages 390-397, XP029924062, ISSN: 1389-1723, DOI: 10.1016/J.JBIOSC.2016.10.011
- WANG XUE ET AL: "Accumulative scFv-Fc antibody gene integration into thehprtchromosomal locus of Chinese hamster ovary cells", JOURNAL OF BIOSCIENCE AND BIOENGINEERING, vol. 124, no. 5, 26 June 2017 (2017-06-26) , pages 583-590, XP085237846, ISSN: 1389-1723, DOI: 10.1016/J.JBIOSC.2017.05.017
- KAWABE YOSHINORI ET AL: "Targeted knock-in of an scFv-Fc antibody gene into the hprt locus of Chinese hamster ovary cells using CRISPR/Cas9 and CRIS-PITCh systems.", JOURNAL OF BIOSCIENCE AND BIOENGINEERING MAY 2018, vol. 125, no. 5, May 2018 (2018-05), pages 599-605, XP002783293, ISSN: 1347-4421
- SEYEDEH HODA JAZAYERI ET AL: "Vector and Cell Line Engineering Technologies Toward Recombinant Protein Expression in Mammalian Cell Lines", APPLIED BIOCHEMISTRY AND BIOTECHNOLOGY ; PART A: ENZYME ENGINEERING AND BIOTECHNOLOGY, vol. 185, no. 4, 3 February 2018 (2018-02-03), pages 986-1003, XP055494794, New York ISSN: 0273-2289, DOI: 10.1007/s12010-017-2689-8
- YUKIKO KOYAMA ET AL: "Stable expression of a heterogeneous gene introduced via gene targeting into the HPRT locus of human fibrosarcoma cells", BIOTECHNOLOGY AND BIOENGINEERING, WILEY, US, vol. 95, no. 6, 1 January 2006 (2006-01-01), pages 1052-1060, XP008154913, ISSN: 0006-3592, DOI: 10.1002/BIT.21058 [retrieved on 2006-06-28]

## Description

### BACKGROUND OF THE INVENTION

### Technical Field

The present invention relates to a recombinant cell which enables efficient production of substances of interest such as protein and the like, a method for producing the cell, and a method for producing a substance of interest such as protein and the like by using the cell.

### Background Art

As a recombinant protein production system, variety of methods using prokaryotes or eukaryotes as a host cell are known. According to the recombinant protein production system using a mammalian cell as a host cell, it is possible to subject proteins derived from higher animals including human to post-translational modifications such as sugar chain addition, folding, and phosphorylation in a manner more similar to those produced *in vivo.*

The post-translational modifications are necessary to reproduce physiological activities, which are inherent to protein, in the recombinant protein. Thus, the protein production system using a mammalian cell as a host cell is used as a production system of recombinant protein used in medicines and the like, which particularly requires such physiological activities.

In order to construct the protein production system, there has been proposed a technique, wherein a gene of interest is integrated specifically into a target gene locus in the cell genome to efficiently obtain a recombinant cell which can express the protein stably for a long period.

An example of the target gene includes a hypoxanthine-guanine phosphoribosyltransferase (hprt) gene. The hprt gene is known as one of the housekeeping genes present on the long arm of X chromosome of humans and the like. Cells after knockout of the hprt gene exhibit resistance to a drug, 6-thioguanine (6TG), and therefore negative selection is performed easily.

It has been reported that, by introducing, via a recombinant vector, a gene of interest into the hprt gene locus of HT1080 cell line derived from human male, recombinant cells were obtained which could express protein stably for a long period in the absence of a selection drug (JP 2007-325571A and Koyama Y. et al. (2006) Biotechnology And Bioengineering, 95, 1052-1060).

Further, some of the present inventors has proposed a method of producing a substance of interest such as protein and the like by preparing mammalian cells having multiple copies of an exogenous gene of interest integrated into the hprt gene locus and by culturing the cells (WO 2009/022656). Further, there has been proposed a technique of applying this method to CHO cells (WO2011/040285).

As far as the present inventors know, the above conventional technologies showed phenomena that, when multiple gene expression units were introduced into the same site of chromosome, the gene introduced did not express itself in an expected level or the expression deteriorated with time.

On the other hand, as a technique for amplifying genes, there has been proposed, for example, a method using a recombinase Cre and a target site of Cre, the target site comprising a spacer region and left and right arm regions arranged respectively on the left and right thereof (JP 2009-189318A; Kameyama, Y. et al., Biotechnol. Bioeng. 105, 1106 (2010); and Inao, T., et al., Journal of Bioscience and Bioengineering 120, 99-106, 2015). This method is called an accumulative gene integration system (AGIS) and it is mentioned that, according to this method, amplification of the gene of interest can be performed on a chromosome of an animal cell and that the cells obtained can be used for mass production of recombinant protein.

### [Citation List]

### [Patent Literature]

[PTL 1] JP 2007-325571A
[PTL 2] WO 2009/022656
[PTL 3] WO2011/040285
[PTL 4] JP 2009-189318A

### [None Patent Literature]

[NPL 1] Koyama Y. et al., Biotechnology and Bioengineering, 95, 1052-1060 (2006)
[NPL 2] Kameyama, Y. et al., Biotechnol. Bioeng. 105, 1106 (2010)
[NPL 3] Inao, T., et al., Journal of Bioscience and Bioengineering 120, 99-106 (2015)

### SUMMARY OF THE INVENTION

We have now found that a system, which enables production of a substance of interest such as recombinant protein and the like more stably for a long period, can be realized by, when introducing multiple exogenous genes of interest, controlling chain lengths and sequences among the expression units of the multiple genes of interest. In particular, the present inventors have found that a strong positive linear correlation is shown between the number of the genes of interest introduced and the expression level. The present invention is based on such findings.

Thus, the object of the present invention is to provide a recombinant cell which enables efficient production of a substance of interest such as protein and the like, a method for producing the cells, and a method for producing the substance of interest by using the cells.

And, a cell according to the present invention is a mammalian cell comprising multiple expression units which comprise an exogenous gene of interest and which are integrated therein, wherein the number of the multiple expression units is 3 or more; and further comprising exogenous gap elements which are provided among the multiple expression units wherein the gap elements have chain lengths of 0.5 kbp or more and 20 kbp or less, and the gap elements are mutually different sequences..

Further, a method for producing the cell according to the present invention is characterized in that the multiple expression units and gap elements are integrated by AGIS.

Furthermore, a method for producing a substance of interest such as protein and the like according to the present invention comprises a step of culturing the cells according to the present invention.

### BRIEF DESCRIPTION OF THE DRAWINGS

Fig. 1 is a schematic diagram of a trastuzumab heavy chain expression vector in Step (1-1), where (1) is a general view of the vector and (2) is a trastuzumab heavy chain expression unit which has been cut out.
Fig. 2 is a schematic diagram of a trastuzumab light chain expression vector in Step (1-2), where (1) is a general view of the vector and (2) is a trastuzumab light chain expression unit which has been cut out.
Fig. 3 is a schematic diagram of a method for preparing a x1 AGIS trastuzumab expression vector (vector 1) in Step (1-3).
Fig. 4 is a schematic diagram of a method for preparing a x2 AGIS trastuzumab expression vector (vector 2) in Step (1-4).
Fig. 5 is a schematic diagram of a x3 AGIS trastuzumab expression vector (vector 3) in Step (1-5).
Fig. 6 is a schematic diagram of a CHO hprt AGIS origin vector in Step (2-1).
Fig. 7A and Fig. 7B illustrate: a schematic diagram of integration of CHO hprt AGIS origin vector into the hprt gene locus of a CHO cell by homologous recombination in Step (2-3); and a result of confirmation of the integration. It is a schematic diagram which illustrates a relationship among a CHO hprt AGIS origin vector, a hprt gene region of a CHO cell, and a DNA which has become an index of genomic PCR of a recombinant CHO cell.
Fig. 7C is a result of electrophoresis for confirmation of vector integration by PCR using genomic DNA as a template.
Fig. 8A and Fig. 8B illustrate: a schematic diagram of integration of vector 1 into a CHO cell by accumulative integration in Step (2-4), the CHO cell having a CHO hprt AGIS origin vector integrated therein; and a result of confirmation of the integration. It is a schematic diagram which illustrates a relationship among vector 1, a CHO hprt AGIS origin vector region of a CHO cell having a CHO hprt AGIS origin vector integrated therein, and a DNA which has become an index of genomic PCR of a recombinant CHO cell.
Fig. 8C is a result of electrophoresis for confirmation of vector integration by PCR using genomic DNA as a template.
Fig. 9A and Fig. 9B illustrate: a schematic diagram of integration of vector 2 into a CHO cell by accumulative integration, the CHO cell having vector 1 integrated therein; and a result of confirmation of the integration. It is a diagram which illustrates a relationship among vector 2, a vector 1 region of a CHO cell having vector 1 integrated therein, and a DNA which has become an index of genomic PCR of a recombinant CHO cell.
Fig. 9C is a result of electrophoresis for confirmation of vector integration by PCR using genomic DNA as a template.
Fig. 10A and Fig. 10B illustrate: a schematic diagram of integration of vector 3 into a CHO cell by accumulative integration, the CHO cell having vector 1 and vector 2 integrated therein; and a result of confirmation of the integration. It is a diagram which illustrates a relationship among vector 3, vector 1 and vector 2 regions of a CHO cell having vector 1 and vector 2 integrated therein, and a DNA which has become an index of genomic PCR of a recombinant CHO cell.
Fig. 10C is a result of electrophoresis for confirmation of vector integration by PCR using genomic DNA as a template.
Fig. 11A illustrates results of quantification of copy numbers of trastuzumab heavy chain and trastuzumab light chain in a CHO cell having vector 1 integrated therein, a CHO cell having vector 1 and vector 2 integrated therein, and a CHO cell having vector 1, vector 2, and vector 3 integrated therein. It illustrates results of quantification of copy numbers of trastuzumab heavy chain DNA and trastuzumab light chain DNA in the chromosome of each cell having a vector(s) integrated therein.
Fig. 11B illustrates results of quantification of copy numbers of trastuzumab heavy chain and trastuzumab light chain in a CHO cell having vector 1 integrated therein, a CHO cell having vector 1 and vector 2 integrated therein, and a CHO cell having vector 1, vector 2, and vector 3 integrated therein. It illustrates results of quantification of copy numbers of trastuzumab heavy chain mRNA and trastuzumab light chain mRNA in the chromosome of each cell having a vector(s) integrated therein.
Fig. 12A is a confirmation result of long-term stability in the antibody expression levels of a CHO cell having vector 1 integrated therein, a CHO cell having vector 1 and vector 2 integrated therein, and a CHO cell having vector 1, vector 2, and vector 3 integrated therein.
Fig. 12B is a result of addition, to Fig. 12A, of data of antibody expression levels by a CHO cell having vector 1, vector 2, and vector 3 integrated therein after subculture for 112 days, 168 days, and 224 days.
Fig. 13 is a schematic diagram of a method for preparing a x2 AGIS gap element A vector in Comparative Example 2.
Fig. 14A and Fig. 14B illustrate: a schematic diagram of integration of a x2 AGIS gap element A vector into a CHO cell by accumulative integration, the CHO cell having vector 1 integrated therein; and a result of confirmation of the integration. It is a diagram which illustrates a relationship among a x2 AGIS gap element A vector, a vector 1 region of a CHO cell having a vector 1 integrated, and a DNA which has become an index for genomic PCR of a recombinant CHO cell.
Fig. 14C is a result of electrophoresis for confirmation of vector integration by PCR using genomic DNA as a template.
Fig. 15 illustrates a confirmation result of long-term stability in antibody expression level of a CHO cell having vector 1 and a x2 AGIS gap element A vector integrated therein.
Fig. 16 is a schematic diagram of a method for preparing a x2 AGIS gap element B500 vector in Example 3.
Fig. 17A and Fig. 17B illustrate: a schematic diagram of integration of a x2 AGIS gap element B500 vector into a CHO cell by accumulative integration, the CHO cell having vector 1 integrated; and a result of confirmation of the integration. It is a diagram which illustrates a relationship among a x2 AGIS gap element B500 vector, a vector 1 region of a CHO cell having vector 1 integrated, and a DNA which has become an index for genomic PCR of a recombinant CHO cell.
Fig. 17C is a result of electrophoresis for confirmation of vector integrationby PCR using genomic DNA as a template.
Fig. 18 is a confirmation result of long-term stability in antibody expression level of a CHO cell having vector 1 and x2 AGIS gap element B500 vector integrated therein.
Fig. 19 is a schematic diagram of a method for preparing a x2 AGIS gap element C500 vector in Example 4.
Fig. 20A and Fig. 20B illustrate: a schematic diagram of integration of a x2 AGIS gap element C500 vector into a CHO cell by accumulative integration, the CHO cell having vector 1 integrated therein; and a result of confirmation of the integration. It is a diagram that illustrates a relationship among a x2 AGIS gap element C500 vector, a vector 1 region of a CHO cell having vector 1 integrated therein, and a DNA which has become an index for genomic PCR of a recombinant CHO cell.
Fig. 20C is a result of electrophoresis for confirmation of vector integration by PCR using genomic DNA as a template.
Fig. 21 is a confirmation result of long-term stability in antibody expression level of a CHO cell having vector 1 and x2 AGIS gap element C500 vector integrated therein.
Fig. 22 is a schematic diagram of a method for preparing a x1 AGIS gap element A500 vector in Example 5.
Fig. 23 is a schematic diagram of a method for preparing a x2 AGIS gap element C500-B500 vector in Example 5.

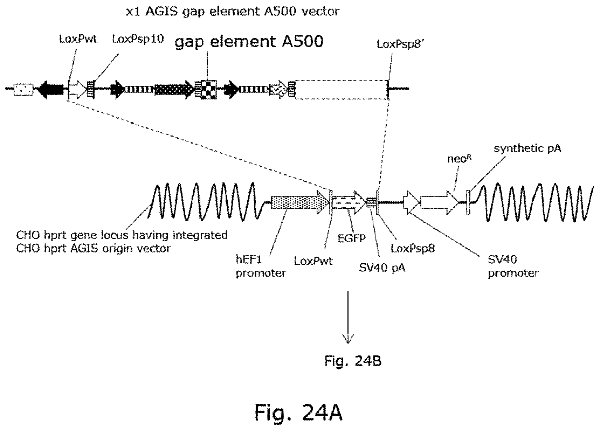

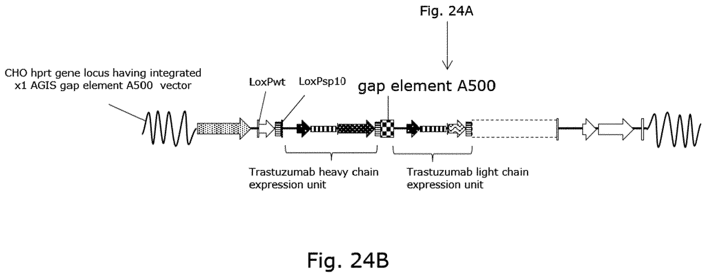

Fig. 24A and Fig. 24B are a schematic diagram of integration of x1 AGIS gap element A500 vector, in Example 5, into a CHO cell by accumulative integration, the CHO cell having a CHO hprt AGIS origin vector integrated therein. It is a diagram that illustrates a relationship between a x1 AGIS gap element A500 vector and a CHO hprt AGIS origin vector region of a CHO cell having CHO hprt AGIS origin vector integrated therein.
Fig. 25A and Fig. 25B illustrate: a schematic diagram of integration of a x2 AGIS gap element C500-B500 vector into a CHO cell by accumulative integration, the CHO cell having x1 AGIS gap element A500 vector integrated therein; and a result of confirmation of the integration. It is a diagram that illustrates a relationship among a x2 AGIS gap element A vector, a x1 AGIS gap element A500 region of CHO cell having x1 AGIS gap element A500 vector integrated therein, and a DNA which has become an index for genomic PCR of a recombinant CHO cell.
Fig. 25C is a result of electrophoresis for confirmation of vector integration by PCR using genomic DNA as a template.
Fig. 26 is a confirmation result of long-term stability in antibody expression level of a CHO cell having x1 AGIS gap element A500 vector and x2 AGIS gap element C500-B500 vector integrated therein.

### DETAILED DESCRIPTION OF THE INVENTION

### Gap element

The "expression unit" according to the present invention, which is integrated into the mammalian cell, is the structure which is not limited as long as the structure enables expression of an exogenous gene of interest, i.e., a substance of interest such as protein, nucleic acid, or the like. In particular, in the case of protein, it preferably refers to a unit provided with a promoter, a coding region of the protein of interest (from the transcription start point to the stop codon), and a poly A sequence. Details of compositional elements of an expression unit such as a gene of interest and the like will be described later.

The "promoter" is a string of DNA sequence having a promoter function, which is obtained from a genomic DNA or a viral genome. Alternatively, the promoter can be a sequence containing a region which has a promoter function, which is obtained from a known DNA construct by cutting out with a suitable restriction endonuclease or by taking out after amplification by a PCR reaction.

The mammalian cell according to the present invention has 3 or more expression units integrated therein and, further, has "exogenous gap elements" among the multiple expression units. In the present invention, the gap elements have a chain length of 0.5 kbp or more, and the gap elements are mutually different sequences. Due to presence of such gap elements among the expression units, there is stably obtained an improvement in expression in proportion to the number of the expression units introduced therein.

While the expression level of the protein of interest is expected to be proportional to the number of genes introduced, in practice, a decrease in the expression level with time has been observed with conventional techniques, even though an expression level proportional to the number of genes is obtained immediately after the introduction. In the present invention, a stable expression level can be maintained for a long period, for example, for 50 days or more, for 100 days or more under preferable conditions, for 200 days under more preferable conditions, and for 300 days under most preferable conditions. The term "stable expression level" means that production relative to that immediately after establishment of the cell is maintained preferably at 70% or more, more preferably at 80% or more, and most preferably at 90% or more.

According to a preferred embodiment of the present invention, the lower limit of chain length of a gap element is preferably 0.5 kbp, more preferably 1 kbp, further more preferably 1.5 kbp, and most preferably 2 kbp. In addition, the upper limit of length of the gap element is preferably 20 kbp, more preferably 10 kbp, further more preferably 5 kbp, and most preferably 3 kbp. A preferable range of chain length of the gap element is determined by selecting and combining the lower limit value and the upper limit value.

According to one embodiment of the present invention, it is advantageous for stable long-term production of recombinant protein that at least one of the multiple gap elements is designed to have a chain length of 1 kbp or more.

Furthermore, in the present invention, the multiple gap elements are so designed as to be mutually different sequences, but mutual homology is designed to be preferably 99.5% or less, more preferably 95% or less, further more preferably 90% or less, further more preferably 87.5% or less, further more preferably 85% or less, further more preferably 82.5% or less, further more preferably 80% or less, further more preferably 77.5% or less, further more preferably 75% or less, further more preferably 72.5% or less, further more preferably 70% or less, further more preferably 67.5% or less, further more preferably 65% or less, further more preferably 62.5% or less, further more preferably 60% or less, further more preferably 57.5% or less, further more preferably 55% or less, further more preferably 52.5% or less, and most preferably 50% or less.

In the present invention, homology is determined in the following manner. That is, of two gap elements one want to compare, homology of an arbitrary sequence in the longer sequence, which has the same length as the full length of the shorter sequence, is determined successively, and the maximum value of homology obtained is defined as the homology. Specific methods for determining homology include a method of using a homology search function of genetic information software, and the like. More specifically, the gap elements can be compared by using (base sequence) × (base sequence) homology search of GENETYX Ver. 10 software (GENETYX CORPORATION).

In the present invention, the gap element is exogenous. The term "exogenous" is used to mean not only a sequence not intrinsically possessed by the mammalian cell into which the gap element is integrated but also a sequence which, even though it is a sequence possessed by the mammalian cell into which the gap element is integrated, should not intrinsically be present at the position.

In the present invention, the gap element does not possess a particular function. In other words, the gap element can be defined as a sequence between two expression units. However, the gap element may contain a sequence having a specific function, as long as it does not intrinsically exert a bad influence on expression of a substance of interest such as protein and the like.

Therefore, according to one embodiment of the present invention, the gap element can be composed of, for example, a sequence arbitrarily obtained from chromosomes of other cells and organisms, a sequence prepared by synthesis based on a sequence designed on a computer, a sequence which constitutes a vector other than the expression unit (such as a simple vector skeleton, a selection marker, a reporter such as GFP, and the like), vector parts remaining as a result of the AGIS (accumulative gene integration system) which will be described later, a marker sequence, and the like.

### Sequence as site of integration into animal cell

In the present invention, the site of integration of the expression unit can be appropriately determined by considering techniques of integrating the expression unit and the like. For example, there can be mentioned a method of integrating the expression unit into a stable gene locus, a method of integrating the expression unit randomly into a suitable site, a method of integrating the expression unit into an extrachromosomal vector, and the like. According to a preferred embodiment of the present invention, the integration technique includes the method of integrating the expression unit into a stable gene locus. Here, the stable gene locus includes, for example, the hypoxanthine-guanine phosphoribosyltransferase (hprt) gene locus, the ROSA 26 gene locus region, the PhiC31 pseudo attP site, and the like. Further, in the case of random insertion, there is mentioned a method of screening the cell having an expression unit integrated incidentally at a stable site on the chromosome. Furthermore, the integration site may not be on the chromosome, but may be, for example, on an artificial chromosome such as human artificial chromosome (HAC vector), mouse artificial chromosome (MAC vector), and the like.

According to a preferred embodiment of the present invention, there is used the hprt gene locus as an integration site. Integration of an expression unit into this target region inactivates functions of the hprt gene by inhibiting transcription and expression of the hprt gene, and is preferable for obtaining a recombinant cell efficiently by negative selection using 6-TG and the like.

Furthermore, in the present invention, the target region can be appropriately determined in the hprt gene locus, as long as it does not interfere with expression of the gene of interest. A specific sequence can be determined with reference to descriptions in WO 2011/040285.

### Mammalian cell

Further, in the present invention, the mammalian cells used as the host cells are preferably one derived from a Chinese hamster, and there can be used a cell line established from cells taken out from an organ thereof such as, for example, lung, ovary, kidney, and the like. Specific examples of such Chinese hamster cells include, for example, CHO cells, CHO-K1, CHO-K1SV (Lonza), CHO-S (Life Technologies), DG44 (Life Technologies), DXB11, CHOZN GS (Merck), CHOZN DHFR (Merck), derivative cell lines of these, and the like.

In addition, as the mammalian cells, also usable is one derived from human, mouse, monkey, and the like. For example, specific examples of cells derived from human include HEK 293, PER.C6 (Crucell), HT 1080, and the like. Further, specific examples of cells derived from the mouse include NS0, BHK, SP2/0, SP2/0-AG14, and the like. As an example of the cells derived from monkey, there can be used COS and the like.

### Gene of interest and expression unit

Furthermore, in the present invention, the gene of interest preferably encodes protein useful as a medicine. The gene, whether it is a sequence derived from cDNA or a structural gene containing a natural intron derived from genomic DNA, can be suitably used. The protein, which the gene encodes, specifically includes antibodies, enzymes, cytokines, hormones, coagulation factors, regulatory proteins, receptors, functional factors, and the like. However, preferable are monoclonal antibodies, polyclonal antibodies, erythropoietins, tissue plasminogen activators (t-PA), granulocyte-colony stimulating factors (G-CSF), and the like.

According to one embodiment of the present invention, a sequence length of the gene which encodes protein is preferably 200 to 8000 bp, more preferably 300 to 3000 bp, and most preferably 400 to 2500 bp.

As for the kind of protein, the protein produced by mammalian cells is sometimes intended to be generated after being subjected to post-translational modifications characteristic of the mammalian cells. In such an embodiment, the protein is preferably a glycoprotein. The glycoprotein specifically includes: monoclonal antibodies such as trastuzumab, rituximab, panitumumab, pertuzumab, obinutuzumab, bevacizumab, tocilizumab, adalimumab, ipilimumab, daratumumab, nivolumab, atezolizumab, pembrolizumab, mogamulizumab, brentuximab, ixekizumab, secukinumab, brodalumab, omalizumab, evolocumab, denosumab, idarucizumab, alirocumab, dinutuximab, reslizumab, alemtuzumab, briakinumab, ocrelizumab, romosozumab, bezlotoxumab, sarilumab, burosumab, benralizumab, sapelizumab, lebrikizumab, farletuzumab, gantenerumab, patritumab, inebilizumab, adecatumumab, tildrakizumab, fasinumab, margetuximab, bococizumab, dupilumab, erenumab, bimagrumab, durvalumab, galcanezumab, risankizumab, andecaliximab, avelumab, vadastuximab, begelomab, suptavumab, depatuxizumab, opicinumab, tanezumab, ocaratuzumab, coltuximab, pamrevlumab, lirilumab, fulranumab, teprotumumab, urelumab, bleselumab, landogrozumab, neihulizumab, foralumab, anrukinzumab, codrituzumab, icrucumab, glembatumumab, ganitumab, amatuximab, ensituximab, namilumab, ponezumab, dectrekumab, isatuximab, claudiximab, olokizumab, mavrilimumab, ficlatuzumab, monalizumab, crenezumab, diridavumab, demcizumab, lenzilumab, seribantumab, blosozumab, ontuxizumab, lodelcizumab, clazakizumab, polatuzumab, simtuzumab, denintuzumab, anetumab, nemolizumab, indatuximab, imalumab, otlertuzumab, tarextumab, lulizumab, tesidolumab, emibetuzumab, abrilumab, brazikumab, emactuzumab, lumretuzumab, mirvetuximab, varlilumab, cabiralizumab, trevogrumab, evinacumab, pidilizumab, rovalpituzumab, rinucumab, sacituzumab, theralizumab, bimekizumab, ublituximab, leucotuximab, vesencumab, ulocuplumab, vandortuzumab, sonepcizumab, atinumab, flanvotumab, brontictuzumab, vantictumab, concizumab, sevacizumab, enoblituzumab, ralpancizumab, necitumumab, ofatumumab, ramucirumab, elotuzumab, natalizumab, vedolizumab, belimumab, eculizumab, palivizumab, epratuzumab, tremelimumab, tralokinumab, etrolizumab, sirukumab, olaratumab, risankizumab, ligelizumab, panobacumab, tisotumab, cetuximab, golimumab, infliximab, siltuximab, ustekinumab, mepolizumab, canakinumab, basiliximab, daclizumab, solanezumab, anifrolumab, gevokizumab, guselkumab, elgemtumab, enfortumab, crotedumab, oleclumab, gerilimzumab, nesvacumab, parsatuzumab, perakizumab, narnatumab, enavatuzumab, sofituzumab, enoticumab, pateclizumab, tovetumab, pinatuzumab, fezakinumab, placulumab, samalizumab, ozanezumab, vatelizumab, ascrinvacumab, dacetuzumab, carlumab, fresolimumab, imgatuzumab, onartuzumab, oxelumab, and the like; antibody fusion proteins such as etanercept, abatacept, belatacept, aflibercept, rilonacept, efraloctocog, eftrenonacog, dulaglutide, atacicept, luspatercept, baminercept, asinercept, dalantercept, sotatercept, ipafricept, ramatercept, asphotase, and the like; recombinant glycoproteins such as erythropoietin, darbepoetin, alteplase, anistreplase, monteplase, tenecteplase, reteplase, octacog, rurioctocog, moroctocog, turoctocog, susoctocog, simoctocog, nonacog, trenonacog, eptacog, lenograstim, imiglucerase, dolnase, agalsidase, laronidase, alglucosidase, velaglucerase, galsulphase, idursulfase, taliglucerase, hyaronidase, elosulfase, follitropin, thyrotropin, cerliponase, and the like; bispecific antibodies such as emicizumab, vanucizumab, istiratumab, pasotuximab, duligotuzumab, duvortuxizumab, and the like; vaccines containing sugar chains and virus like particles (VLP); or the like.

Further, proteins having no sugar chain include: ScFv antibody, Fab antibody, and modified or fused proteins thereof; functional factors such as ATF4, ATF6, VAMP8, Snap 23, and the like; reporters such as GFP, DsRed, CFP, YFP, SEAP, and the like; and receptors such as EGFR, VEGF, PDGF and the like, vaccines containing no sugar chain, virus-like particles, and the like.

Furthermore, in the present invention, the gene of interest preferably encodes: functional nucleotides such as antisense, siRNA, aptamer, miRNA antisense, miRNA mimic, decoy, CpG oligo, and the like; and functional nucleotides to be fused with proteins to constitute nucleotide fusion proteins.

Also, in the present invention, the expression unit is preferably integrated as an expression unit containing elements required for the expression such as a promoter sequence, a transcription terminator, and the like. Therefore, according to one embodiment of the present invention, the expression unit contains at least a promoter sequence and a transcription terminator.

In addition, the promoter and the transcription terminator can be determined appropriately depending on the kind, nature, and the like of the gene of interest, and suitable examples of such a promoter sequence include, for example, CHEF1α promoter, hEF1 promoter, CMV promoter, SV40 promoter, CAG promoter, CAGG promoter, ribosomal protein promoter, heat shock protein promoter, U6 promoter, tetracycline-inducible promoter, chloramphenicol-inducible promoter, and the like. Also, suitable examples of the transcription terminator include, for example, BGH poly-A signal sequence, SV40 poly-A signal sequence, and the like.

Further, as elements required for the expression other than the promoter sequence and the transcription terminator, there can be used one by appropriately selecting from, for example, regulatory elements for efficiently expressing the gene of interest, which include, for example, enhancer, IRES (internal ribosome entry site) sequence, UCOE, STAR, MAR, Power express element, and the like. The regulatory element can be arranged depending on its nature at a suitable site in the expression unit. These elements required for the expression are preferably selected in consideration of productivity and the like of the substance of interest such as protein and the like.

According to one embodiment of the present invention, different genes of interest can be integrated in one expression site. It is a remarkable advantage of the present invention that such expression of multiple kinds of genes of interest can be produced stably for a long period.

### Integration of multiple expression units and gap elements

In the present invention, the method for introducing the expression unit and gap element is not limited as long as the method can integrate a plurality of them. However, there can be mentioned a method of introducing a plurality of them in stages and a method of introducing a plurality of them simultaneously.

The method for introducing the expression units and gap elements in stages include, for example, a method of using a recombinase such as Cre, ϕC31, R4, Flp, and the like, and a method of using a genome editing technology represented by TALEN, CRISPR/Cas9, and the like. According to a preferred embodiment of the method of using Cre recombinase, the method is preferably performed by accumulative gene integration system (AGIS) as disclosed by Japanese Patent Literature 2009-189318.

The method for introducing multiple expression units and gap elements simultaneously includes, for example, a method where full length of these are chemically synthesized and introduced.

### AGIS (Accumulative Gene Integration System)

This method enables repeated integration of a gene of interest into a chromosome by using recombinase Cre and variant LoxP sequences. In other words, this is a method which enables amplification of the gene of interest on the chromosome. This method uses the fact that, by combining characteristics of both arm mutation and spacer mutation of LoxP, an independently irreversible insertion reaction occurs Cre-dependently, and the method is so designed that the target site which can be integrated returns to an initial state after two integration reactions. Herewith, it becomes possible, in principle, to repeat the gene integration reaction infinitely. Specifically, the method is:
a gene recombination method for performing two or more recombinations on a chromosome, comprising use of recombinase Cre and a target site of Cre comprising a spacer region, and a left arm region and a right arm region arranged on the left and right thereof, respectively,
the method comprising the steps of providing:
   (A) target site 1 comprising spacer region 1, one arm region having a mutation, and the other arm region which is a wild type, the target site being integrated into a host chromosome;
   (B) an integration cassette comprising gene 1, and target site 2 and target site 3 which are linked respectively to both sides of gene 1, wherein the target site 2 comprises the spacer region 1, one arm region having a mutation on a side opposite to the target site 1 and close to the gene 1, and the other arm region which is a wild type, and the target site 3 comprises spacer region 2 which can function independently of the spacer region 1, one arm region having a mutation on the same side as the target site 1 and close to the gene 1, and the other arm region which is a wild type; and
   (C) a substitution cassette comprising gene 2, and target site 4 and target site 5 which are linked respectively to both sides of the gene 2, wherein the target site 4 comprises the spacer region 2, one arm region having a mutation on a side opposite to the target site 1 and close to the gene 2, and the other arm region which is a wild type, and the target site 5 comprises spacer region 3 (where the spacer region 3 can function in the same manner as the spacer region 1 or independently of each of the spacer regions 1 and 2) , one arm region having a mutation on the same side as the target site 1 and close to the gene 2, and the other arm region which is a wild type;
and steps of:
   (1) reacting the target site 1 and the integration cassette by action of Cre to obtain a reaction product; and
   (2) reacting the reaction product and the substitution cassette by action of Cre.

In the above, preparation of the "(A) target site 1 comprising spacer region 1, one arm region having a mutation, and the other arm region on other side which is a wild type, the target site being integrated into a host chromosome," that is, integration of an integration origin into the chromosome can be performed by: (1) a method of introducing the integration origin at a specific site of an animal cell by homologous recombination using, for example, a homology arm vector; (2) a method of introducing the integration origin at a specific site by a gene editing technology; (3) a method of screening the cell having the integration origin introduced incidentally at a suitable site by random insertion.

### Vector

In the present invention, the multiple expression units and gap elements are integrated into the vector and thereby can be integrated into a mammalian cell genome. Such a vector system includes plasmid vector, cosmid vector, phage vector, artificial chromosome vector, or the like.

The vector can be constructed suitably by combining a restriction endonuclease cleavage reaction and a ligation reaction. For example, the vector can be constructed by: having restriction endonuclease recognition sequences contained at both ends of each constituent unit; performing a cleavage reaction with a restriction endonuclease for the recognition sequence; removing unnecessary DNA sequences (sequence for engineering in E. coli, and the like) by a treatment such as gel excision and the like; and performing a ligation reaction of the units obtained.

A vector DNA constructed by the ligation reaction is purified by extraction with phenol-chloroform and the like, and can be propagated in a host cell such as E. coli, yeast, and the like selected in consideration of the kind of the vector and the like.

### Transfection of vector

As a method for introducing the vector, there can suitably be utilized generally used methods. For example, there can be mentioned calcium phosphate transfection, electroporation, microinjection, a DEAE-dextran method, a method of using a liposome reagent, lipofection using a cationic lipid, a method of using a virus vector, and the like. Here, when the vector is of a cyclic form, it can be linearized by a publicly-known method before being introduced into a cell.

Furthermore, depending on the nature of the vector and the substance of interest such as protein and the like, and in consideration of acquisition efficiency of the recombinant cells and the like, there can also be appropriately applied: a site-specific integration system utilizing a recombinase such as Cre/LoxP system, Flp/FRT system, and the like; or a method of using genome editing technology represented by virus-derived integrase, virus vector, TALEN, CRISPR/Cas9, and the like. Such embodiments are also included in the present invention.

### Selection of cell line

Further, in the production method according to the present invention, it is preferable to perform cell selection after the transfection. The selection step enables selection of cells with high accuracy by utilizing a method of using a marker gene, a method of selecting the cells with the expression level of the gene of interest or gene product as an index, and the like.

Also, in the production method according to present invention, after obtaining the recombinant cells, they can be cultured even under a serum-free condition by acclimatizing them to a Chemical Defined medium and the like. Such an acclimatization condition can be appropriately determined depending on the state of the recombinant cells.

### Production method of substance of interest

Further, according to other embodiment of the present invention, there is provided a method for producing a substance of interest such as protein and the like, comprising preparing the recombinant cells and culturing the cells to produce the substance of interest such as protein and the like. According to the method above, the substance of interest such as protein and the like can be obtained efficiently and stably.

As a medium in the culturing step, there can be appropriately selected a publicly known medium depending on the state of the recombinant cells, but the medium is preferably a serum-free medium. Further, the medium is preferably one having no selection drug added in consideration of culturing cost and purification cost. A preferable example of the medium includes a Chemical Defined medium and the like.

As the culturing method, there can be applied a publicly known method such as a batch culture method, a fed-batch culture method, a perfusion culture method, and the like.

### EXAM PLES

The present invention will be described in detail by reference to the following examples, but the present invention is not limited to these examples.

In the following examples, LoxP sequences used were as follows.

| Name | Base Sequence (5'→3') | | | |
|---|---|---|---|---|
| *LoxPwt* | ATAACTTCCTATA | GCATACAT | TATACGAAGTTAT | (SEQ ID NO: 73) |
| *LoxPsp8* | ATAACTTCGTATA | aagtatcc | TATACGAAcggta | (SEQ ID NO: 74) |
| *LoxPsp8'* | taccgTTCGTATA | aagtatcc | TATACGAAGTTAT | (SEQ ID NO: 75) |
| *LoxPsp10* | ATAACTTCGTATA | accataat | TATACGAAcggta | (SEQ ID NO: 76) |
| *LoxPsp10'* | taccgTTCGTATA | accataat | TATACGAAGTTAT | (SEQ ID NO: 77) |

### Preparation and Evaluation of Cells in Examples 1 and 2 and Comparative

### Example 1

### Step 1: Preparation of Accumulatively Integration Vector Containing Gap Elements of Mutually Different Sequences among Expression Units

### Step (1-1) Preparation of Trastuzumab Heavy Chain Expression Vector

To a secretion signal peptide (METDTLLLWVLLLWVPGSTGD) (SEQ ID NO: 1) of mouse Igκ was bound the amino acid sequence of trastuzumab heavy chain (obtained from trastuzumab (gene recombinant) examination report published on Jan. 16, 2008 by Pharmaceuticals and Medical Devices Agency), and a DNA sequence which encodes this sequence was designed. Next, on the 5' side of the sequence were attached the recognition sequence (underlined part) for restriction endonuclease EcoRI (GAATTCgccgccacc) and a Kozak sequence (lowercase letters), and to the 3' terminal were attached a stop codon and the recognition sequence (tgaCTCGAG) for restriction endonuclease Xhol. DNA was synthesized based on this sequence to prepare a trastuzumab heavy chain gene fragment.

Subsequently, using pmaxGFP plasmid (amaxa) as a template, SV40 poly A was amplified by a PCR reaction with a SV40pA-s/SV40pA-a primer set to obtain an SV40 poly A fragment. To the 5' terminal of the sense primer was attached the recognition sequence (underlined part) for restriction endonuclease Xhol, and to the 5' terminal of the antisense primer, was attached the recognition sequence (underlined part) for restriction endonuclease Clal.
SV40pA-s primer:
   5'-CTCGAGctcgatgagtttggacaaac-3' (SEQ ID NO: 2)
SV40pA-a primer:
   5'-ATCGATGTCGACAAGCTTTCTAGAACGCGTtaccacatttgtagaggtt-3' (SEQ ID NO: 3)

Next, the genomic DNA of CHO-K1 (ATCC CCL-61) was extracted with MAG Extractor (TOYOBO) and, using this as a template, a PCR reaction with a CHEF1α promoter-s/CHEF1α promoter-a primer set was performed to amplify a CHO endogenous EF1a promoter and to obtain a CHEF1α promoter fragment. To the 5' terminal of the sense primer was attached the recognition sequence (underlined part) for restriction endonuclease Clal, and to the 5' terminal of the antisense primer was attached the recognition sequence (underlined part) for restriction endonuclease EcoRI.
CHEF1α promoter-s primer:
   5'-ATCGATGTCGACAAGCTTTCTAGAACGCGTccacacaatcagaaccaca-3' (SEQ ID NO: 4)
CHEF1α promoter-a primer:
   5'-GAATTCgcggtggttttcacaacacc-3' (SEQ ID NO: 5)

Then, using pCR-Bluntll-TOPO vector (Life Technologies) as a template, a kanamycin resistance gene, a zeocin resistance gene, and a replication origin were amplified by a PCR reaction with a pUC-s/pUC-a primer set to obtain the plasmid backbone. To each of the 5'-terminal of the sense primer and the 5'-terminal of the antisense primer was attached the recognition sequence (underlined part) for restriction endonuclease Clal.
pUC-s primer:
   5'-ATCGATGCTTACATGGCGATAGCTAG-3' (SEQ ID NO: 6)
pUC-a primer:
   5'-ATCGATGAATCAGGGGATAACGCAGG-3' (SEQ ID NO: 7)

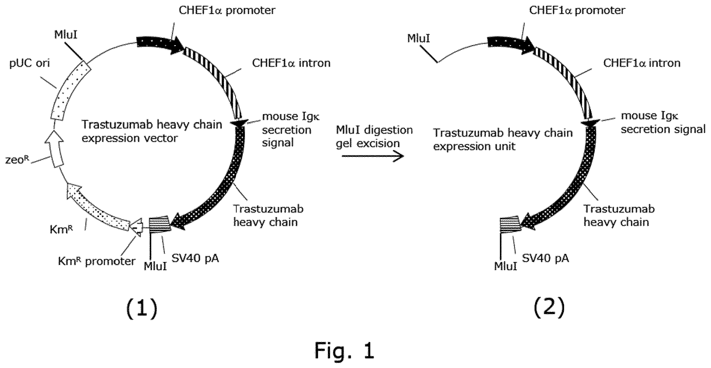

The prepared and obtained CHEF1α promoter fragment, trastuzumab heavy chain gene fragment, SV40 poly A fragment, and plasmid backbone were linked by restriction endonuclease digestion and ligation of the terminals to prepare the trastuzumab heavy chain expression vector of Fig. 1 (1).

The trastuzumab heavy chain expression vector obtained was digested with restriction endonuclease Mlul and subjected to agarose gel electrophoresis, and the trastuzumab heavy chain expression unit of Fig. 1 (2) was extracted.

### Step (1-2) Preparation of Trastuzumab Light Chain Expression Vector

In the same manner as in Step (1-1), to the secretion signal peptide of mouse Igκ was bound the amino acid sequence of trastuzumab light chain (obtained from the same examination report as in Step (1-1)), and a DNA sequence which encodes this sequence was designed. Next, on the 5' side of the sequence were attached the recognition sequence (underlined part) (GAATTCgccgccacc) for restriction endonuclease EcoRI and a Kozak sequence (lowercase letters), and to the 3' terminal were attached a stop codon (lowercase letters) and the recognition sequence (underlined part) (tgaCTCGAG) for restriction endonuclease Xhol. DNA was synthesized based on this sequence to prepare a trastuzumab light chain gene fragment.

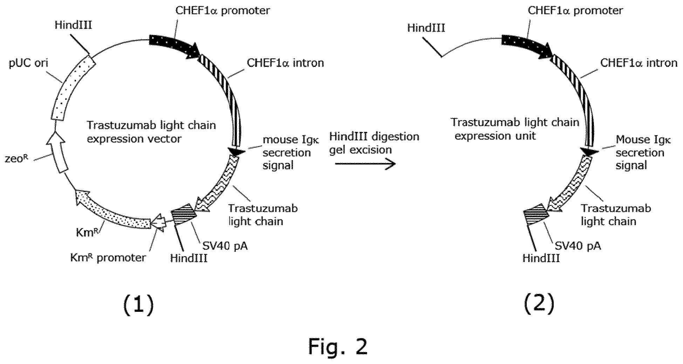

The CHEF1α promoter fragment, SV40 poly A fragment, and plasmid backbone, which were obtained and prepared in Step (1-1), and the trastuzumab light chain gene fragment were linked by restriction endonuclease digestion and ligation of the terminals to prepare the trastuzumab light chain expression vector of Fig. 2 (1).

The thus obtained trastuzumab light chain expression vector was digested with restriction endonuclease Mlul and subjected to agarose gel electrophoresis, and the trastuzumab light chain expression unit of Fig. 2 (2) was extracted.

### Step (1-3) Preparation of x1 AGIS Trastuzumab Expression Vector (Vector 1)

Using pQBI25 plasmid vector (Wako Pure Chemical Industries, Ltd.) as a template, a PCR reaction was performed with a pUC-amp-s1/pUC-amp-a1 primer set to obtain a fragment (1) containing a replication origin and ampicillin resistance gene. To the 5' terminal of the sense primer were attached the recognition sequence (underlined part) for restriction endonuclease Spel and LoxPsp8' sequence (lowercase letters) of Table 1, and to the 5' terminal of the antisense primer was attached the recognition sequence (underlined part) for restriction endonuclease BamHI.
pUC-amp-s1 primer:
   5'-ACTAGTCtaccgttcgtataaagtatcctatacgaagttatTAGCTTCAGTGAGCAAAAG-3' (SEQ ID NO: 8)
pUC-amp-a1 primer:
   5'-GGATCCtcgcgtaggtggcacttttc-3' (SEQ ID NO: 9)

Subsequently, using pBApo-EF1α Pur vector (TaKaRa) as a template, a PCR reaction was performed with a puro-s/puro-a primer set to obtain a fragment containing puromycin resistance gene and SV40 poly A. To the 5' terminal of the sense primer were attached the recognition sequence (underlined part) for restriction endonuclease BamHI and LoxPwt sequence (lowercase letters) of Table 1, and to the 5' terminal of the antisense primer were attached the recognition sequence (underlined part) for restriction endonuclease Spel and a complementary sequence (lowercase letters) to the LoxPsp10 sequence of Table 1.
puro-s primer:
   5'-GGATCCataacttcgtatagcatacattatacgaagttatCCACCGAGTCAAGCCCACGGT-3' (SEQ ID NO: 10)
puro-a primer:

The fragment (1) containing a replication origin and ampicillin resistance gene and the fragment containing puromycin resistance gene and SV40 poly A, which were obtained above, were ligated to prepare x1 basic vector.

Next, using genomic DNA of human embryonic kidney cell-derived HEK293 (JCRB ID: 9068) as a template, a dummy sequence of 3130 bp was amplified with a dummy sequence-s/dummy sequence-a primer set. To the 5' terminal of the sense primer was attached the recognition sequence (underlined part) for restriction endonuclease Hindlll, and to the 5' terminal of the antisense primer was attached the recognition sequence (underlined part) for restriction endonuclease Spel.
Dummy sequence-s primer:
   5'-AAGCTTGGCAACATAGCGAGACTTCG-3' (SEQ ID NO: 12)
Dummy sequence-a primer:
   5'-ACTAGTGTGTACAGAAGGCTGAAAGG-3' (SEQ ID NO: 13)

Furthermore, using genomic DNA of HEK293 as a template, gap element A (SEQ ID NO: 14) of 2051 bp was PCR amplified with a gap element A-s/gap element A-a primer set, and the length of amplification product was confirmed by agarose gel electrophoresis. To the 5' terminal of the sense primer was added the recognition sequence (underlined part) for restriction endonuclease Mlul, and to the 5' terminal of the antisense primer was added the recognition sequence (underlined part) for restriction endonuclease Hindlll.
Gap element A-s primer:
   5'-ACGCGTTGAAAGTGTGAGTTCCGGAG-3' (SEQ ID NO: 15)
Gap element A-a primer:
   5'-AAGCTTAACTGCAGCCTATTGGTAGC-3' (SEQ ID NO: 16)

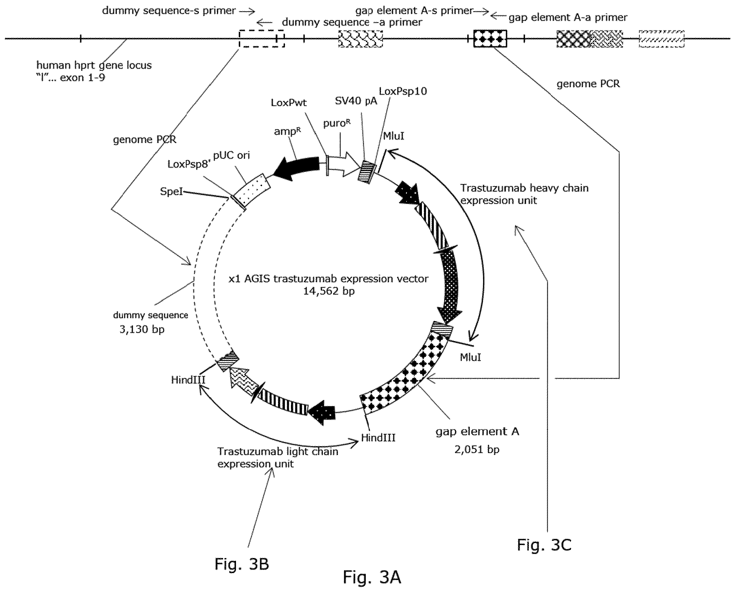

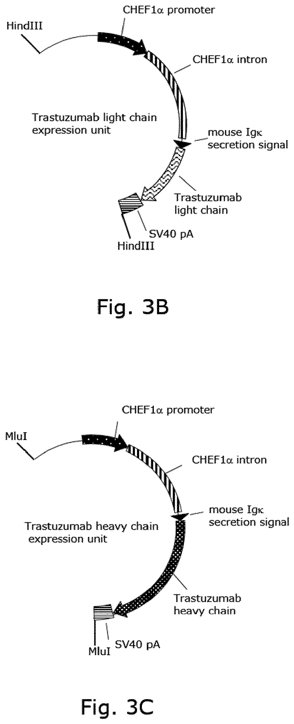

The gap element A was inserted between the Mlul and Hindlll sites of the x1 basic vector obtained above. Then, the dummy sequence was inserted between the Hindlll and Spel sites, and subsequently the fragment of the trastuzumab heavy chain expression unit of Step (1-1) was inserted in the Mlul site. Finally, the fragment of the trastuzumab light chain expression unit of Step (1-2) was inserted in the Hindlll site to prepare the vector 1 shown in Fig. 3.

### Step (1-4) Preparation of x2 AGIS Trastuzumab Expression Vector (Vector 2)

Using pQBI25 plasmid vector as a template, a PCR reaction was performed with a pUC-amp-s2/pUC-amp-a2 primer set to obtain a fragment (2) containing a replication origin and an ampicillin resistance gene. To the 5' terminal of the sense primer were attached the recognition sequence (underlined part) for restriction endonuclease Spel and LoxPsp10' sequence (lowercase letters) of Table 1, and to the 5' terminal of the antisense primer was attached the recognition sequence (underlined part) for restriction endonuclease BamHI.
pUC-amp-s2 primer:
   5'-ACTAGTCtaccgttcgtataaccataattatacgaagttatTAGCTTCATGTGAGCAAAAG-3' (SEQ ID NO: 17)
pUC-amp-a2 primer:
   5'-GGATCCtcgcgTaggtggcacttttc-3' (SEQ ID NO: 18)

Subsequently, using pcDNA3.1/Hygro (+) vector (Life Technologies) as a template, a PCR reaction was performed with a Hygro-s/Hygro-a primer set to obtain a fragment containing a hygromycin resistance gene and SV40 poly A. To the 5' terminal of the sense primer were attached the recognition sequence (underlined part) for restriction endonuclease BamHI and LoxPwt sequence (lowercase letters) of Table 1, and to the 5' terminal of the antisense primer were attached the recognition sequence (underlined part) for restriction endonuclease Spel and a complementary sequence (lowercase letters) to LoxPsp8 sequence of Table 1.
Hygro-s primer:
   5'-GGATCCataacttcgtatagcatacattatacgaagttatCCAAAAAGCTGAACTCACCGC-3' (SEQ ID NO: 19)
Hygro-a primer:

The fragment (2) containing a replication origin and ampicillin resistance gene and the fragment containing hygromycin resistance gene and SV40 poly A, which were obtained above, were ligated to prepare a x2 basic vector.

Next, using genomic DNA of HEK293 as a template, gap element C of 2045 bp was PCR amplified with a gap element C-s/gap element C-a primer set, and the length of amplification product was confirmed by agarose gel electrophoresis. To the 5' terminal of the sense primer was attached the recognition sequence (underlined part) for restriction endonuclease Mlul, and to the 5' terminal of the antisense primer was attached the recognition sequence (underlined part) for restriction endonuclease HindIII.
gap element C-s primer:
   5'-ACGCGTCGTTTTCTCACTGGCTGCTG-3' (SEQ ID NO:21)
gap element C-a primer:
   5'-AAGCTTACTCTCATAGTACCTGTCCC-3' (SEQ ID NO:22)

Further, using genomic DNA of HEK293 as a template, gap element B (SEQ ID NO: 23) of 3093 bp was PCR amplified with a gap element B-s/gap element B-a primer set, and the length of amplification product was confirmed by agarose gel electrophoresis. To the 5' terminal of the sense primer was attached the recognition sequence (underlined part) for restriction endonuclease Hindlll, and to the 5' terminal of the antisense primer was attached the recognition sequence (underlined part) for restriction endonuclease Spel.
gap element B-s primer:
   5'-AAGCTTGCTCATTGACACTCTGTACC-3' (SEQ ID NO: 24)
gap element B-a primer:
   5'-ACTAGTCTCACGTGGAGCAGATAAGC-3' (SEQ ID NO: 25)

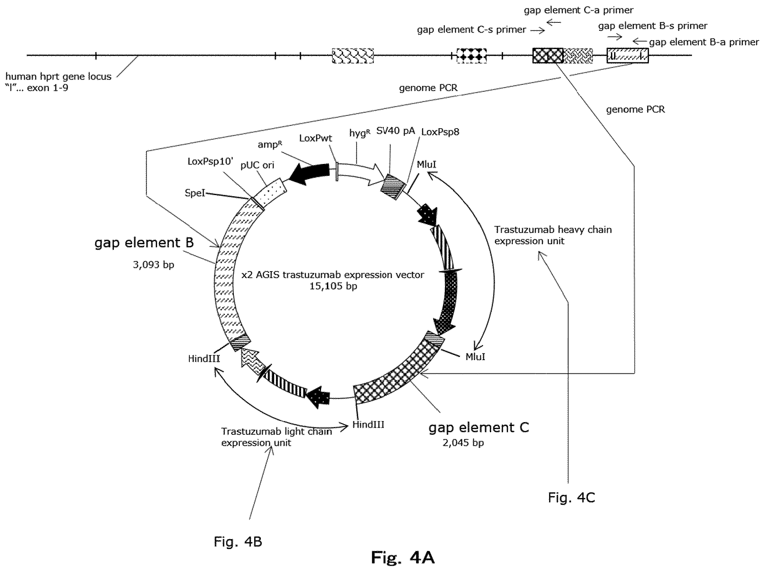

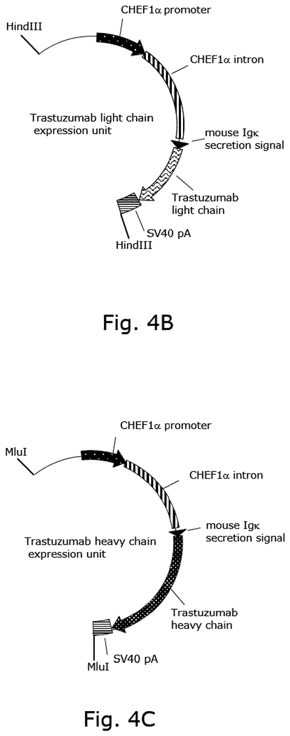

Gap element C (SEQ ID NO. 26) was inserted between the Mlul and Hindlll sites of the x2 basic vector obtained above. Then, the gap element B was inserted between the Hindlll and Spel sites, and subsequently the fragment of the trastuzumab heavy chain expression unit of Step (1-1) was inserted in the Mlul site. Finally, the fragment of the trastuzumab light chain expression unit of Step (1-2) was inserted in the Hindlll site to prepare the vector 2 shown in Fig. 4.

### Step (1-5) Preparation of x3 AGIS Trastuzumab Expression Vector (Vector 3)

Using pQBI25 plasmid vector as a template, a PCR reaction was performed with a pUC-amp-s3/pUC-amp-a3 primer set to obtain a fragment (3) containing a replication origin and ampicillin resistance gene. To the 5' terminal of the sense primer were attached the recognition sequence (underlined part) for restriction endonuclease Spel and LoxPsp8' sequence (lowercase letters) of Table 1, and to the 5' terminal of the antisense primer was attached the recognition sequence (underlined part) for restriction endonuclease BamHI.
pUC-amp-s3 primer:
   5'-ACTAGTCtaccgttcgtataaagtatcctatacgaagttatTAGCTTCATGTGAGCAAAAG-3' (SEQ ID NO: 27)
pUC-amp-a3 primer:
   5'-GGATCCtcgcgTaggtggcacttttc-3' (SEQ ID NO: 28)

Subsequently, using pcDNA6/V5-His/lacZ vector (Life Technologies) as a template, a PCR reaction was performed with a Bsd-s/Bsd-a primer set to obtain a fragment containing blasticidin resistance gene and SV40 poly A. To the 5' terminal of the sense primer were attached the recognition sequence (underlined part) for restriction endonuclease BamHI and LoxPwt sequence (lowercase letters) of Table 1, and to the 5' terminal of the antisense primer were attached the recognition sequence (underlined part) for restriction endonuclease Spel and a complementary sequence (lowercase letters) to the LoxPsp10 sequence of Table 1.
Bsd-s primer:
   5'-GGATCCataacttcgtatagcatacattatacgaagttatCCGCCAAGCCCTGTCCCAGGA-3' (SEQ ID NO: 29)
Bsd-a primer:

The fragment (3) containing a replication origin and ampicillin resistance gene and the fragment containing blasticidin resistance gene and SV40 poly A, which were obtained above, were linked to prepare x3 basic vector.

Next, using genomic DNA of HEK293 as a template, gap element E of 2033 bp was PCR amplified with a gap element E-s/gap element E-a primer set, and the length of amplification product was confirmed by agarose gel electrophoresis. To the 5' terminal of the sense primer was attached the recognition sequence (underlined part) for restriction endonuclease Mlul, and to the 5' terminal of the antisense primer was attached the recognition sequence (underlined part) for restriction endonuclease HindIII.
gap element E-s primer:
   5'-ACGCGTCAACCTACTGTGGCATAGAC-3' (SEQ ID NO: 31)
gap element E-a primer:
   5'-AAGCTTACAGGCAGAGGTTATCCTGG-3' (SEQ ID NO: 32)

Further, using genomic DNA of HEK293 as a template, gap element D (SEQ ID NO: 33) of 3001 bp was PCR amplified with a gap element D-s/gap element D-a primer set, and the length of amplification product was confirmed by agarose gel electrophoresis. To the 5' terminal of the sense primer was attached the recognition sequence (underlined part) for restriction endonuclease Hindlll, and to the 5' terminal of the antisense primer was attached the recognition sequence (underlined part) for restriction endonuclease Spel.
gap element D-s primer:
   5'-AAGCTTAGATCACAGCTCACTGTGGC-3' (SEQ ID NO: 34)
gap element D-a primer:
   5'-ACTAGTCGGCTGACACCTGTAATCTC-3' (SEQ ID NO: 35)

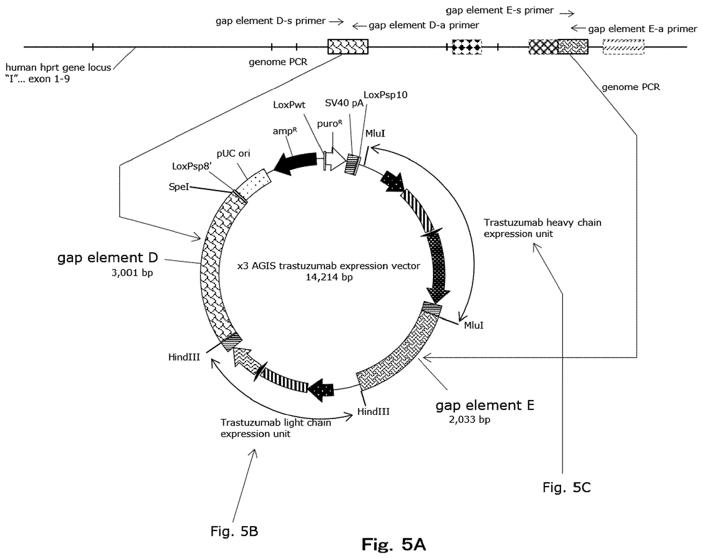

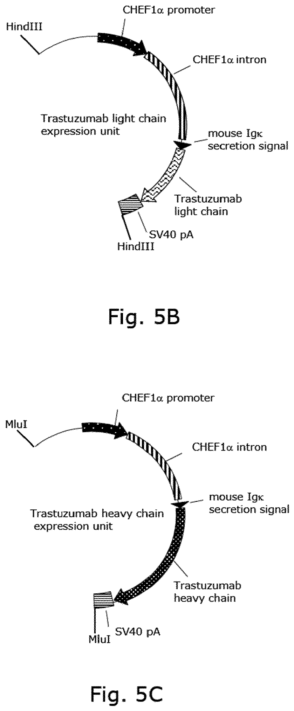

Gap element E (SEQ ID NO. 36) was inserted between the Mlul and HindIII sites of the x3 basic vector obtained above. Then, the gap element D was inserted between Hindlll and Spel sites, and subsequently the fragment of the trastuzumab heavy chain expression unit of Step (1-1) was inserted in the Mlul site. Finally, the fragment of the trastuzumab light chain expression unit of Step (1-2) was inserted in the HindIII site to prepare the vector 3 shown in Fig. 5.

### Step 2: Accumulative Integration of Accumulative integration Vector into CHO hprt Gene Locus, the Vector Containing Gap Elements Having Mutually Different Sequences between Expression Units

### Step (2-1) Preparation of CHO hprt AGIS Origin Vector

Using genomic DNA of HEK293 as a template, hEF1 promoter sequence was amplified with an hEF1-s/hEF1-a primer set. To the 5' terminal of the sense primer was attached the recognition sequence (underlined part) for restriction endonuclease Mlul, and to the 5' terminal of the antisense primer was attached a phosphate group (P).
hEF1-s primer:
   5'-ACGCGTagccgaacctctcgcaagtc-3' (SEQ ID NO: 37)
hEF1-a primer:
   5'P-TTTGGCTTTTAGGGGTAGTTTTCACGACAC-3' (SEQ ID NO: 38)

Next, using as a template a vector containing EGFP gene sequence possessed by TOTO Ltd. and SV40 poly A sequence, EGFP gene sequence+SV40 poly A sequence was amplified with an EGFP-s/EGFP-a primer set. To the 5' terminal of the sense primer were attached a phosphate group (P) and LoxPwt sequence (lowercase letters) of Table 1, and to the 5'terminal of the antisense primer were attached the recognition sequence (underlined part) for restriction endonuclease Xbal and the complimentary chain (lowercase letters) for LoxPsp8 sequence of Table 1.
EGFP-s primer:
   5'P-ATGataacttcgtatagcatacattatacgaagttatCCATGGTGAGCAGGGCGAGGA-3' (SEQ ID NO: 39)
EGFP-a primer:
   5'-TCTAGAtaccgttcgtataggatactttatacgaagttatTACCACATTTGTAGAGGTTT-3' (SEQ ID NO: 40)

Then, the hEF1 promoter sequence and the PCR amplification product of EGFP gene sequence+SV40 poly A sequence were ligated to prepare a fragment of hEF1 promoter+EGFP+SV40 poly A.

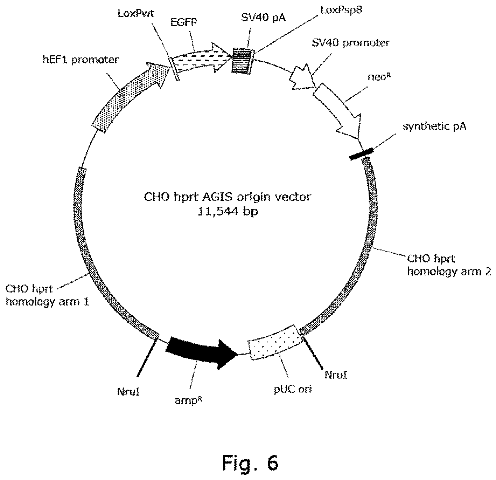

Subsequently, the CHO 2.5kbx2 heavy chain vector described in WO 09/022656 was digested by restriction endonucleases Mlul and Xbal, a fragment of 3.1 kbp was removed, and the hEF1 promoter+EGFP+SV40 poly A fragment prepared in the preceding paragraph was inserted to prepare the CHO hprt AGIS origin vector shown in Fig. 6.

### Step (2-2) Preparation of Cre Expression Plasmid

Using 705-Cre cm, Expression Vector (Funakoshi Co., Ltd.) as a template, a PCR reaction was performed with a Cre-s/Cre-a primer set to amplify Cre recombinase gene. To the 5' terminal of sense primer was attached the recognition sequence (underlined part) for restriction endonuclease Kpnl, and to the 5'terminal of the antisense primer was attached the recognition sequence (underlined part) for restriction endonuclease BgIII (underlined part).
Cre-s primer:
   5'-GGTACCGAAGCCGCTAGCGCTACCGGTCGCCACCatgtccaatttactgccgtac-3' (SEQ ID NO: 41)
Cre-a primer:
   5'-AGATCTctaatcgccatcttccagca-3' (SEQ ID NO: 42)

Next, pmaxGFP plasmid was digested by restriction endonucleases Kpnl and BgIII, the GFP sequence was removed, and the amplification product of the Cre recombinase gene was inserted to prepare a Cre expression plasmid.

### Step (2-3) Integration of CHO hprt AGIS Origin Vector into CHO hprt Gene Locus

A cell line was prepared by adaptation CHO-K1 (ATCC CCL-61) to serum-free suspension culture. This cell line was cultured in AMEM medium (composition: Advanced MEM (Life Technologies), 5% (v/v) FBS (Life Technologies), and 1xGlutaMAX (Life Technologies)) under conditions of 37°C and 8% CO₂ for 2 days.

The cultured cells were detached with Trypsin/EDTA (Life Technologies) and, after termination of the reaction with AMEM medium, were centrifuged. The cell pellet was washed with PBS (Wako Pure Chemical Industries, Ltd.), thereafter centrifuged, and suspended with PBS. The cell suspension in PBS was subjected to cell density measurement and 2×10⁶ cells were centrifuged. The pellet obtained was suspended in Nucleofection T solution (amaxa) and, thereafter, mixed with 2 µg of linearized CHO hprt AGIS origin vector. To the mixture obtained was applied a voltage by Nucleofector II (amaxa) program U-023.

The cells to which a voltage had been applied were suspended in 5 mL of AMEM medium, seeded on a 96-well microplate under serial dilution, and cultured under conditions of 37°C and 8% CO₂ After 2 days from transfection, thereto was added G418 (Life Technologies) (final concentration: 500 µg/mL). After 4 more days, G418 (final concentration: 500 µg/mL) and 6-TG (final concentration: 50 µM) (Wako Pure Chemical Industries, Ltd.) were added and the cells were cultured for further 6 days.

After culture, all wells were checked and G418/6TG resistant colonies were separated. The clones separated were cultured in the AMEM medium to which G418 and 6TG had been added, and the culture was expanded from a 96-well plate to a 24-well palate, then to a 6-well plate, and finally to a 100 mm dish.

Next, each clone was released with Trypsin/EDTA and, after termination of the reaction with the AMEM medium, centrifuged. The cell pellet obtained was washed with CD OptiCHO medium (composition: CD OptiCHO (Life Technologies) and 1×GlutaMax (Life Technologies)), thereafter centrifuged again, and suspended in the CD OptiCHO medium. The cell suspension was subcultured in a 125 ml flask under conditions of 37°C, 8% CO₂, and 128 rpm.

Genomic DNA of a positive clone obtained was extracted with MAG Extractor (TOYOBO). The genomic DNA extracted was analyzed by genomic PCR using a primer set of primer 1/primer 2 and a primer set of primer 3/primer 4.
primer 1: 5'-GACACATGCAGACAGAACAG-3' (SEQ ID NO: 43)
primer 2: 5'-AACTTCTCGGGGACTGTGGG-3' (SEQ ID NO: 44)
primer 3: 5'-CTATCGCCTTCTTGACGAGT-3' (SEQ ID NO: 45)
primer 4: 5'-GTTTGCTAACACCCCTTCTC-3' (SEQ ID NO: 46)

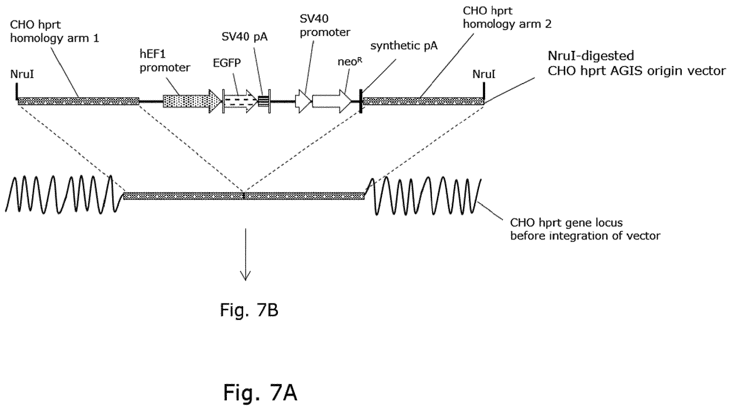

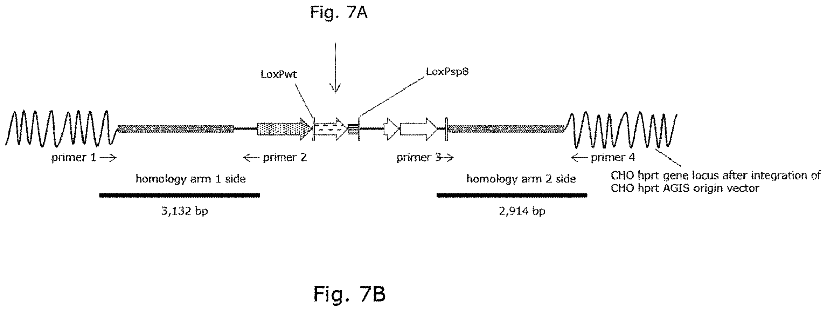

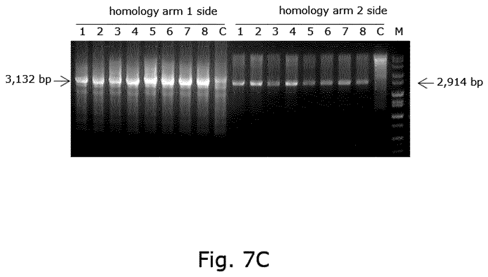

As shown in Fig. 7B, in the case of a correct homologous recombinant clone, a PCR product of 3132 bp is amplified on the homology arm 1 side. Further, a PCR product of 2914 bp is amplified on the homology arm 2 side. The result of analysis was as shown in Fig. 7C. In all of 8 clones analyzed, the amplification fragment of 3132 bp was amplified on the homology arm 1 side and the amplification fragment of 2914 bp was amplified on the homology arm 2 side. Thus, clone No. 1 was used in the following experiment as a cell line having the CHO hprt AGIS origin vector integrated therein.

### Step (2-4) Integration of Vector 1 into CHO hprt Gene Locus

Into the cell line having the CHO hprt AGIS origin vector integrated therein, prepared in Step (2-3), 2 µg of vector 1 and 0.2 µg of Cre expression plasmid were introduced by electroporation as in Step (2-3), and the cell line was seeded on a 96-well plate.

To exchange culture medium after 2 days and 6 days from seeding, and for expanded culturing, there was used AMEM medium+puromycin (final concentration: 12 µg/mL) (Life Technologies).

Genomic DNA of a positive clone obtained was extracted with MAG Extractor. The genomic DNA extracted was analyzed by genomic PCR using a primer set of primer 5/primer 6 and a primer set of primer 7/primer 8.
primer 5: 5'-GGGAGCTCAAAATGGAGGAC-3' (SEQ ID NO: 47)
primer 6: 5'-GAAGAGTTCTTGCAGCTCGG-3' (SEQ ID NO: 48)
primer 7: 5'-GCTTGCATTGTATGTCTGGC-3' (SEQ ID NO: 49)
primer 8: 5'-GGTTCTTTCCGCCTCAGAAG-3 (SEQ ID NO: 50)

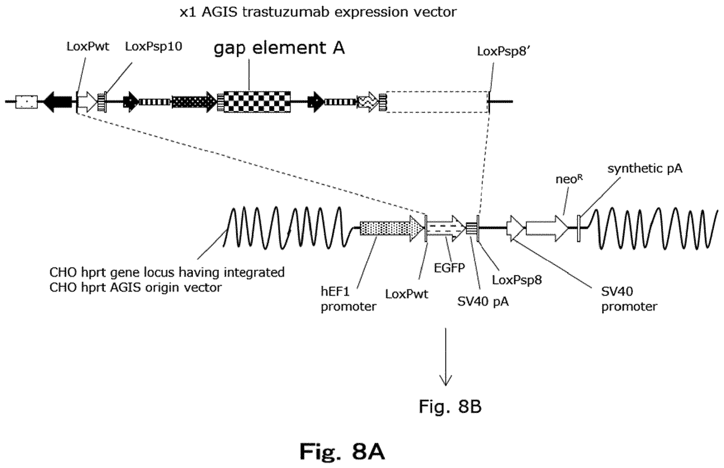

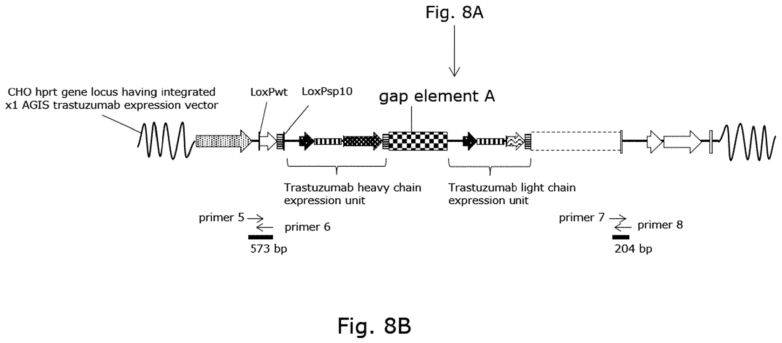

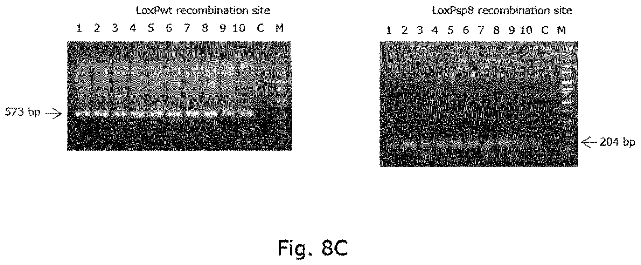

As shown in Fig. 8B, in the case of a correct accumulatively integrated clone, a PCR product of 573 bp is amplified in the vicinity of LoxPwt recombination site. Further, a PCR product of 204 bp is amplified in the vicinity of LoxPst8 recombination site. The result of analysis was as shown in Fig. 8C. In 10 clones out of 10 clones analyzed, the amplification fragment of 573 bp was amplified in the vicinity of LoxPwt recombination site and the amplification fragment of 204 bp was amplified in the vicinity of LoxPst8 recombination site. Thus, clone No. 6 was used in the following experiment as a cell line having the vector 1 integrated therein.

### Step (2-5) Accumulative Integration of Vector 2 into Vector 1-Integrated Cell Line: Preparation of Cell Example 1

Into the cell line having the vector 1 integrated therein, prepared in Step (2-4), 2 µg of vector 2 and 0.2 µg of Cre expression plasmid were introduced by electroporation as in Step (2-3), and the cell line was seeded on a 96-well plate.

To exchange culture medium after 2 days and 6 days from seeding, and for expanded culturing, there was used AMEM medium+Hygromycin (final concentration: 500 µg/mL) (Life Technologies).

Genomic DNA of a positive clone obtained was extracted with MAG Extractor. The genomic DNA extracted was analyzed by genomic PCR using a primer set of primer 9/primer 10 and a primer set of primer 11/primer 12.
primer 9: 5'-GGGAGCTCAAAATGGAGGAC-3' (SEQ ID NO: 51)
primer 10: 5'-GCAATAGGTCAGGCTCTCGC-3' (SEQ ID NO: 52)
primer 11: 5'-GAATGGGTTGGCGGAGCTAC-3' (SEQ ID NO: 53)
primer 12: 5'-GCTGTGGTTCTGATTGTGTGG-3' (SEQ ID NO: 54)

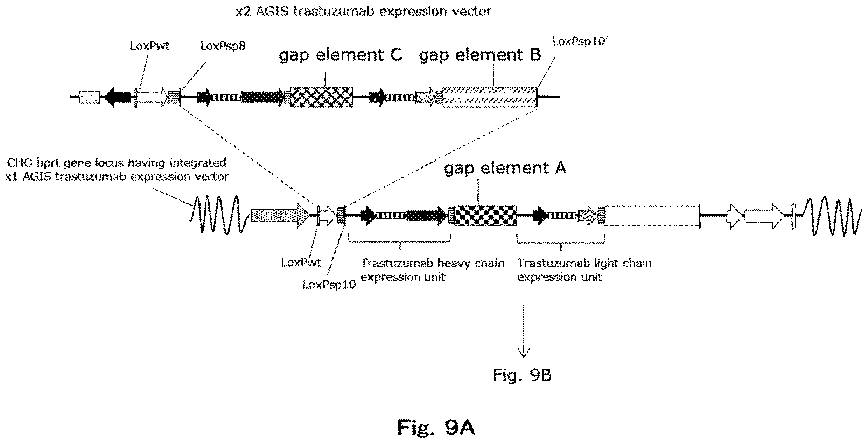

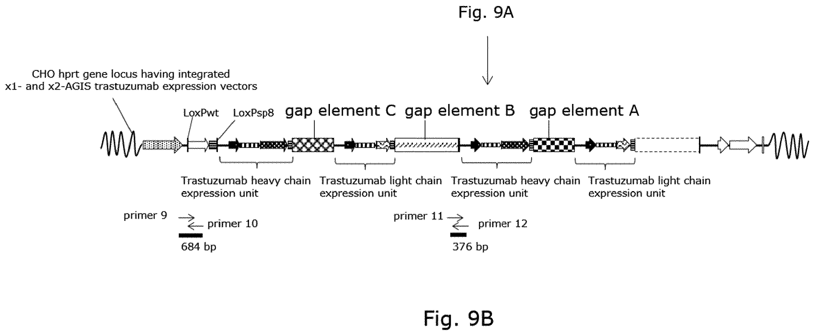

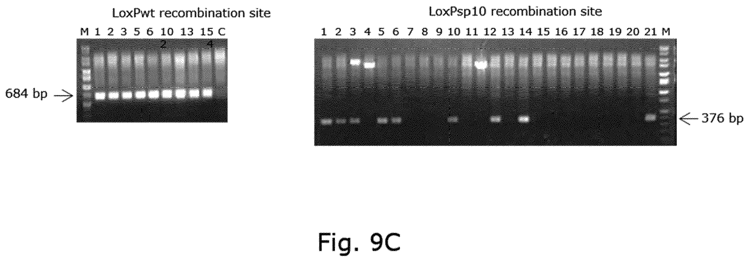

As shown in Fig. 9B, in the case of a correct accumulatively integrated clone, a PCR product of 684 bp is amplified in the vicinity of LoxPwt recombination site. Further, a PCR product of 376 bp is amplified in the vicinity of LoxPsp10 recombination site. The result of analysis was as shown in Fig. 9C. When analysis of the vicinity of LoxPsp10 was performed, in 10 clones out of 24 clones, the amplification product of 376 bp was amplified. When the 10 clones were analyzed in the vicinity of LoxPwt recombination site, in 10 clones out of the 10 clones, the amplification product of 684 bp was amplified. Thus, clone No. 1 was used in the following experiment as a cell line (Cell Example 1) having the vector 1 and vector 2 integrated therein.

### Step (2-6) Accumulative Integration of Vector 3 into Vector 1- and Vector 2-Integrated Cell Line: Preparation of Cell Example 2

Into the cell line having the vector 1 and vector 2 integrated therein, prepared in Step (2-4), 2 µg of vector 3 and 0.2 µg of Cre expression plasmid were introduced by electroporation as in Step (2-4), and the cell line was seeded on a 96-well plate.

To exchange culture medium after 2 days and 6 days from seeding, and for expanded culturing, there was used AMEM medium+Blasticidin (final concentration: 12 µg/mL)(Life Technologies).

Genomic DNA of a positive clone obtained was extracted with MAG Extractor. The genomic DNA extracted was analyzed by genomic PCR using a primer set of primer 13/primer 14 and a primer set of primer 15/primer 16.
primer 13: 5'-GGGAGCTCAAAATGGAGGAC-3' (SEQ ID NO: 55)
primer 14: 5'-GCTGTGGTTCTGATTGTGTGG-3' (SEQ ID NO: 56)
primer 15: 5'-GACTACAAGTGCGTGCCATC-3' (SEQ ID NO: 57)
primer 16: 5'-GCTGTGGTTCTGATTGTGTGG-3' (SEQ ID NO: 58)

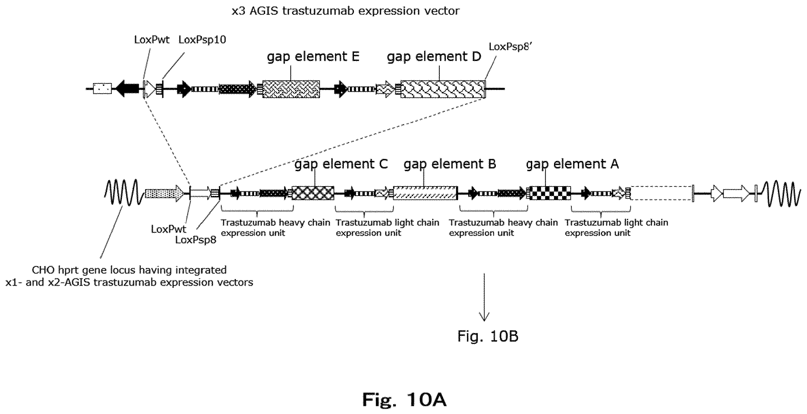

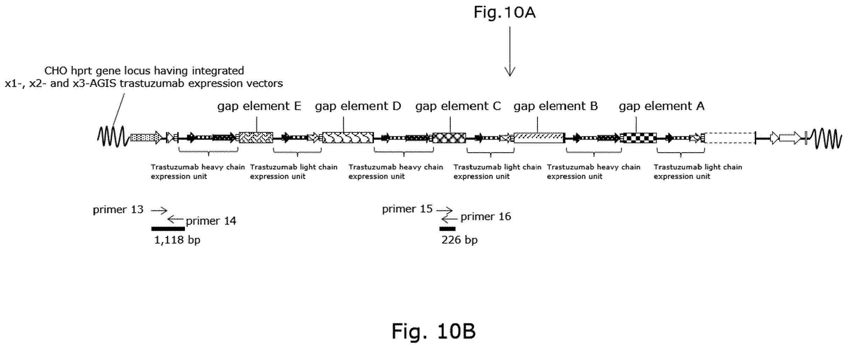

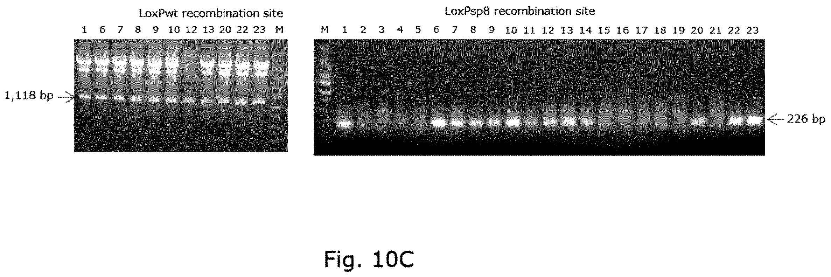

As shown in Fig. 10B, in the case of a correct accumulatively integrated cell, a PCR product of 1118 bp is amplified in the vicinity of LoxPwt recombination site. Further, a PCR product of 226 bp is amplified in the vicinity of LoxPsp8 recombination site. The result of analysis was as shown in Fig. 10C. When analysis of the vicinity of LoxPsp8 was performed, in 11 clones out of 23 clones, the amplification product of 226 bp was amplified. When the 11 clones were analyzed in the vicinity of LoxPwt recombination site, in 11 clones out of the 11 clones, the amplification product of 1118 bp was amplified. Thus, clone No. 22 was used in the following evaluation as a cell line (Cell Example 2) having the vector 1, vector 2, and vector 3 integrated therein.

### Evaluation: Evaluation of Accumulative Integration Vector-Integrated CHO, the Vector Containing Gap Elements Having Mutually Different Sequences Introduced among Expression Units

### Evaluation 1: Measurement of Homology of Gap Elements

Homology between gap elements of the vector 1, vector 2, and vector 3, prepared in Steps (1-3), (1-4), and (1-5), respectively, was measured as follows.

Electronic files of the gap elements were read into GENETYX Ver. 10 software, and "Nucleotide vs Nucleotide Homology" of the "Homology" command was selected.

Next, in the "Parameter" dialogue, checks were entered into both of "Original Sequence" and "Complement Sequence," and the values of "Unit Size" and "Scores alignments" were respectively set to "6" and "100".

Then, electronic files of the gap elements to be compared were added to "Target Sequence" in the "Sequence" dialogue, and "OK" was selected for execution.

Of the values of "Identity (%)," the output measurement results, the highest value was employed as the value of homology.

As a result, homology between respective gap elements was 51% for gap element A-gap element B, 45% for gap element A-gap element C, 48% for gap element A-gap element D, 45% for gap element A-gap element E, 44% for gap element B-gap element C, 52% for gap element B-gap element D, 46% for gap element B-gap element E, 44% for gap element C-gap element D, 45% for gap element C-gap element E, and 45% for gap element D-gap element E.

### Evaluation 2: Expression Level Analysis of Trastuzumab Heavy Chain and Light Chain in Vector 1-Integrated Cell Line (Comparative Example 1), Vector 1- and Vector 2-Integrated Cell Line (Example 1), and Vector 1-, Vector 2-, and Vector 3-Integrated Cell Line (Example 2)

Genomic DNAs of the vector 1-integrated cell line (hereinafter called "Comparative Example 1"), the vector 1- and vector 2-integrated cell line (hereinafter called "Example 1"), and the vector 1-, vector 2-, and vector 3-integrated cell line (hereinafter called Example 2) were extracted with MAG Extractor, the cell lines having been prepared in Steps (2-4), (2-5), and (2-6). The copy numbers of trastuzumab heavy chain and light chain in the extracted genomic DNAs were quantified by real-time PCR.

Quantification of the copy number of the trastuzumab heavy chain was performed with a primer set of heavy chain forward primer/heavy chain reverse primer, and a heavy chain TaqMan probe.
heavy chain forward primer:
   5'-ACGAGGACCCTGAAGTGAAGTTC-3' (SEQ ID NO: 59)
heavy chain reverse primer:
   5'-TGTTCCTCTCTAGGCTTGGTCTTG-3' (SEQ ID NO: 60)
heavy chain TaqMAN probe:
   5'-TACGTGGACGGCGTGGA-3' (SEQ ID NO: 61)

Quantification of the copy number of the trastuzumab light chain was performed with a primer set of light chain forward primer/light chain reverse primer, and a light chain TaqMan probe.
light chain forward primer:
   5'-TCTACTCCGCCTCCTTCCTGTA-3' (SEQ ID NO: 62)
light chain reverse primer:
   5'-TGGCAGTAGTAGGTGGCGAAGT-3' (SEQ ID NO: 63)
light chain TaqMan probe:
   5'-ATCCGGCACCGACTT-3' (SEQ ID NO: 64)

As a real-time PCR instrument, there was used 7300 Real-Time PCR (Applied Biosystems). The quantified values were standardized by the copy number of CHO endogenous GAPDH.

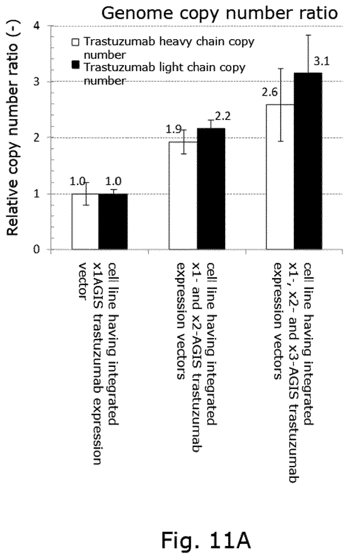

The quantification results were as shown in Fig. 11A. The copy numbers of the trastuzumab heavy chain gene and the trastuzumab light chain gene were in agreement with the copy number of each introduced.

Next, the cells of Comparative Example 1, Example 1, and Example 2 were adjusted in the CD OptiCHO medium to 3×10⁵ cells/mL and were cultured in a 125 mL flask for 2 days under conditions of 37°C, 8% CO₂, and 128 rpm.

Culture solutions of the previous paragraph were centrifuged, the cell pellets were washed with PBS (TaKaRa), mRNA was extracted from 5×10⁴ cells with TaqMan Gene Expression Cells-to-CT Kit (Life Technologies), and cDNA was synthesized by using the Kit. Copy numbers of trastuzumab heavy chain and trastuzumab light chain in the prepared cDNA were quantified by using a primer set of heavy chain forward primer/heavy chain reverse primer, and a heavy chain TaqMAN probe. The quantified values were standardized by the copy number of mRNA CHO endogenous GAPDH, and the relative copy number was calculated by setting that of a cell line to 1.0, in which cell line the copy number of trastuzumab gene introduced was 1.

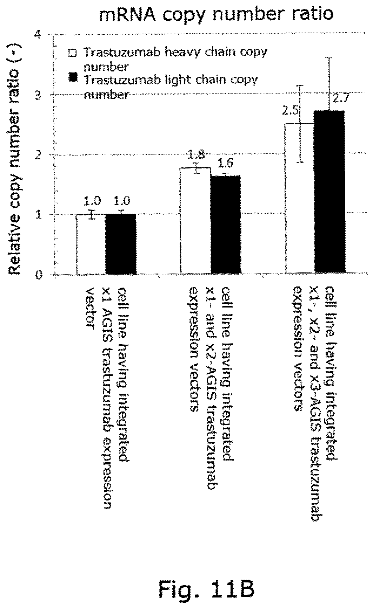

The quantification results were as shown in Fig. 11B. The relative copy numbers of trastuzumab heavy chain mRNA were 1.0, 1.8, and 2.5 respectively in cell lines in which copy numbers of introduced trastuzumab heavy chain genes were 1, 2, and 3, and increased nearly in proportion to the copy number of genes.

Also, as shown in Fig. 11B, the relative copy numbers of trastuzumab light chain mRNA were 1.0, 1.6, and 2.7 respectively in cell lines in which copy numbers of introduced trastuzumab light chain genes were 1, 2, and 3, and increased nearly in proportion to the copy number of genes.

### Evaluation 2: Analysis of Trastuzumab Productivity and Long-Term Expression Stability of Cells of Comparative Example 1, Example 1, and Example 2

The cells of Comparative Example 1, Example 1, and Example 2 were each maintained by subculture in a 125 mL flask under conditions of the CD OptiCHO medium, 37°C, 8% CO₂, and 128 rpm, and an amount of trastuzumab produced by each cell line was timely measured.

Measurement of the amount of trastuzumab produced was performed as follows. The cell line immediately after establishment or in the course of subculture was adjusted in the CD OptiCHO medium to 2×10⁵ cells/mL and was cultured in a 125 mL flask for 8 days under conditions of 37°C, 8% CO₂, and 128 rpm. The cell density of culture solution on the 8th day of culture was measured, and the culture solution was centrifuged to obtain a culture supernatant. The amount of trastuzumab in the culture supernatant was measured by using Biosensor/Protein A Tray (ForteBIO) on the Blitz system (ForteBIO).

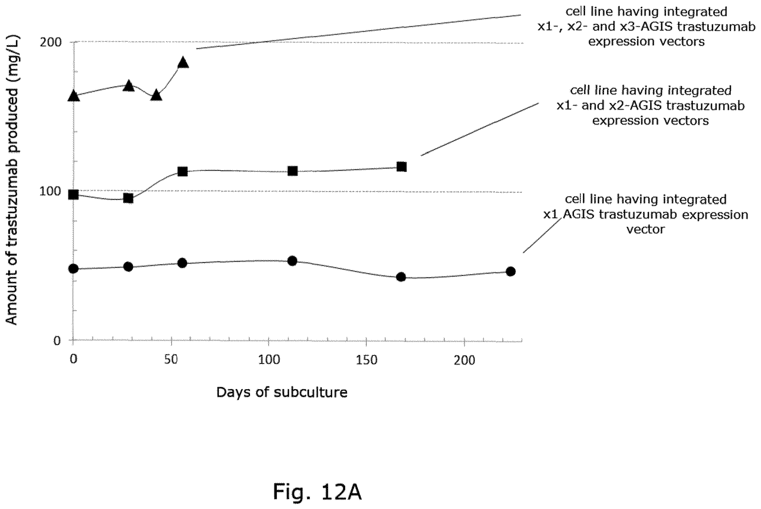

The expression level of trastuzumab in batch culture of Comparative Example 1 was 48.0 mg/L immediately after establishment. Further, during subculture for 224 days, the expression level was stably maintained (Fig. 12A).

The expression level of trastuzumab in batch culture of Example 1 was 97.1 mg/L immediately after establishment. The expression level increased to 113 mg/L by 56 days of subculture, and thereafter was stably maintained at 116 mg/L during 168 days of subculture (Fig. 12A).

The expression level of trastuzumab in batch culture of Example 2 was 164 mg/L immediately after establishment. Further, during subculture for 56 days, the expression level was stably maintained (Fig. 12A).

In the cells of Comparative Example 1, Example 1, and Example 2, the expression levels of trastuzumab relative to the copy number of introduced trastuzumab gene showed a strong positive linear correlation. Further, there occurred no decrease in the expression level due to subculture, and long-term, stable expression was confirmed (Fig. 12A).

Furthermore, the cells of Example 2 were maintained by subculture and, in the same manner as above, the amounts of trastuzumab produced after subculture for 112 days, 168 days, and 224 days were measured, and compared with the amounts in the cells of Comparative Example 1 and Example 1 on these days. Thereat, in order to reduce the quantification error due to day to day variation as much as possible, the culture supernatants which had been stored in the above tests, the results of which are shown in Fig. 12A, were subjected to remeasurement of expression levels of trastuzumab therein. That is, with regard to Comparative Example 1, the culture supernatants sampled immediately after establishment and 28 days, 56 days, 112 days, 168 days, and 224 days thereafter, and stored at -80°C were subjected simultaneously to remeasurement of the expression level of trastuzumab. Similarly, with regard to Example 1, the culture supernatants sampled immediately after establishment and 28 days, 56 days, 112 days, 168 days, and 224 days thereafter, and stored at -80°C were subjected simultaneously to remeasurement of the expression level of trastuzumab. Furthermore, with regard to Example 2, the expression levels of trastuzumab before the above-mentioned days were also remeasured simultaneously in consideration of the quantification error, using the culture supernatants sampled immediately after establishment and 28 days and 56 days thereafter, and stored at -80°C.

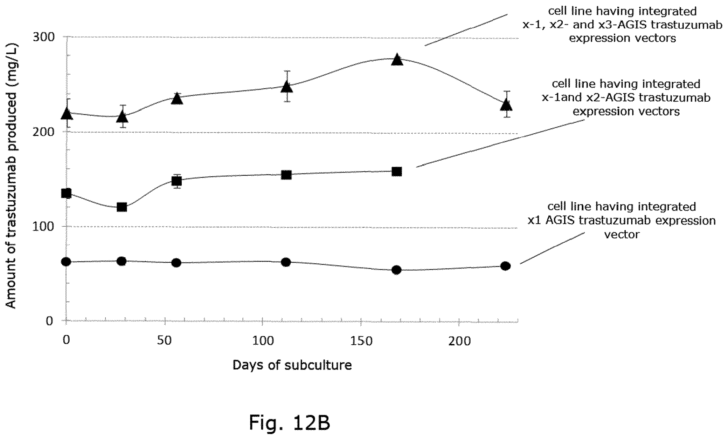

As a result of remeasurement, the expression level of trastuzumab in batch culture of Comparative Example 1 was 62.3 mg/L. In addition, the expression level was stably maintained after subculture of 224 days (Fig. 12B).

As a result of remeasurement, the expression level of trastuzumab in batch culture of Example 1 was 135 mg/L immediately after establishment. The expression level increased to 148 mg/L by 56 days of subculture and, thereafter, was stably maintained at 159 mg/L during subculture of 168 days (Fig. 12B).

As a result of remeasurement, the expression level of trastuzumab in batch culture of Example 2 was 220 mg/L immediately after establishment. In addition, the expression level was stably maintained during subculture of 224 days (Fig. 12B).

### Comparative Example 2: Example of Cell in which Homology between Mutually Different Gap Elements is 100%

A "x2 AGIS gap element A vector," which has as a gap element the same sequence as the full length of gap element A, was prepared as follows.

A sequence of gap element A' (SEQ ID NO: 65) was obtained by conducting artificial gene synthesis. In this sequence, the recognition sequence (AAGCTT) for restriction endonuclease HindIII is attached to the 5' terminal, and the recognition sequence (ACTAGT) for restriction endonuclease Spel is attached to the 3' terminal. Further, the sequence of 2043 bp just before the Spel at 3' terminal is completely the same as the sequence contained in 2051 bp of the gap element A of Step (1-3). On the other hand, 1 kbp after HindIII recognition sequence at 5' terminal is the same as the gap element B of Step (1-4).

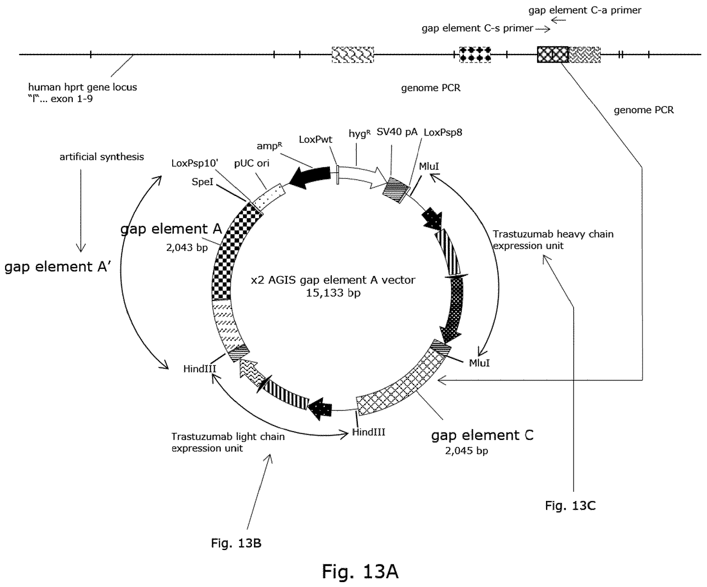

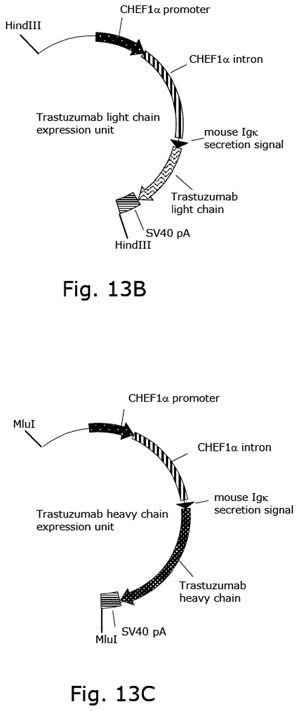

The gap element C was inserted between the Mlul and Hindlll sites of the x2 basic vector obtained in Step (1-4). Then, the gap element A was inserted between the Hindlll and Spel sites, and subsequently the fragment of the trastuzumab heavy chain expression unit of Step (1-1) was inserted in the Mlul site. Finally, the fragment of the trastuzumab light chain expression unit of Step (1-2) was inserted in the Hindlll site to prepare the x2 AGIS gap element A vector shown in Fig. 13.

Next, the x2 AGIS gap element A vector was accumulatively integrated into the vector 1-integrated cell line as follows to prepare the cells.

The x2 AGIS gap element A vector was integrated into the vector 1-integrated cell line, prepared in Step (2-4), in the same manner as in Step (2-3) to obtain a positive clone.

Genomic DNA of the positive clone obtained was extracted with MAG Extractor. The genomic DNA extracted was analyzed by genomic PCR using a primer set of primer 9/primer 10 and a primer set of primer 16/primer 12.
primer16: 5'-TCCCAAAGTACCGGGATTAC-3' (SEQ ID NO: 66)

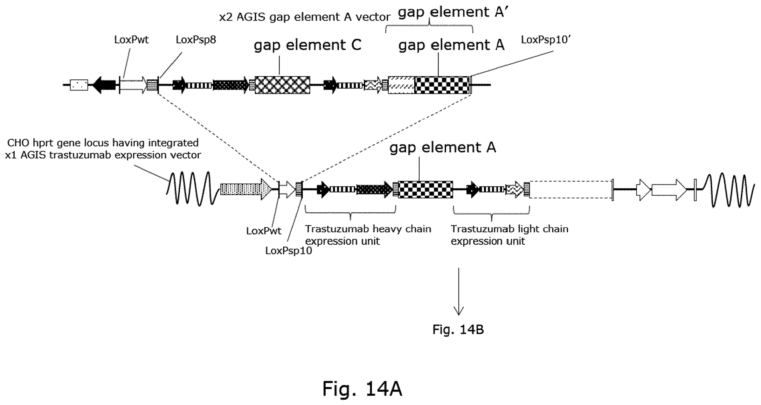

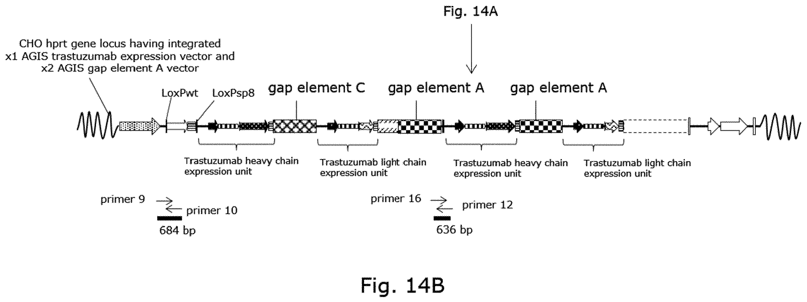

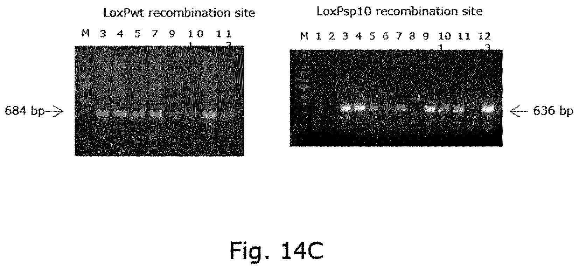

As shown in Fig. 14B, in the case of a correctly accumulatively integrated clone, a PCR product of 684 bp is amplified in the vicinity of LoxPwt recombination site. Further, a PCR product of 636 bp is amplified in the vicinity of LoxPsp10 recombination site. The result of analysis was as shown in Fig. 14C. When analysis of the vicinity of LoxPsp10 recombination site was performed, in 8 clones out of 13 clones, the product of 636 bp was amplified. When the 8 clones were analyzed in the vicinity of LoxPwt recombination site, in 8 clones out of 10 clones, the amplification product of 684 bp was amplified.

Clone No. 3 was selected and, in the same manner as in Evaluation 1, the copy numbers of the heavy chain and light chain of trastuzumab in the genomic DNA was quantified by real-time PCR in the same manner as in Evaluation 1.

As a result, the copy number of trastuzumab heavy chain was 1.9±0.1 and the copy number of light chain was 1.9±0.1, which were approximately in agreement with 2, the number of genes introduced. Thus, clone No. 3 was used in the following evaluation as vector1- and x2 AGIS gap element A vector-introduced cell line (hereinafter, this cell is called "Comparative Example 2").

### Evaluation: Evaluation of Vector-Integrated CHO Cell Line, the Vector Having Gap Elements of Mutually Homologous Sequences Introduced between Expression Units

### Evaluation 3: Analysis of Expression Levels of Trastuzumab Heavy Chain and Light Chain in Cells of Comparative Example 2

The cells of Comparative Example 2 were maintained by subculture in the same manner as in Evaluation 2, and the production amount of trastuzumab in the cell line was timely measured.

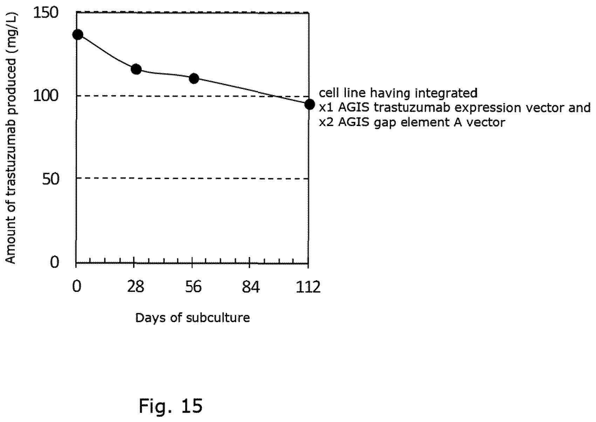

The expression level of trastuzumab in batch culture of Comparative Example 2 was 137 mg/L immediately after establishment. The expression level decreased with continuation of subculture, and after 112 days of subculture, the expression level decreased to 95.5 mg/L (Fig. 15).

### Example 3: Example of Cell Having One Gap Element of 0.5 kbp (#1) Example of Cell in which Gap Element B of Example 1 is Changed to Chain Length of 0.5 kbp

A "x2 AGIS Gap Element B 500 Vector" having one gap element of 541 bp was prepared as follows.

Using genomic DNA of HEK293 as a template, a gap element B500 (SEQ ID NO: 67) of 541 bp was PCR amplified with a gap element B500-s/gap element B-a primer set, and the length of amplification product was confirmed by agarose gel electrophoresis. To the 5' terminal of the sense primer was attached the recognition sequence (underlined part) for restriction endonuclease HindIII.
gap element B500-s primer: 5'-AAGCTTaatctttgtcagcagttccc-3' (SEQ ID NO: 68)

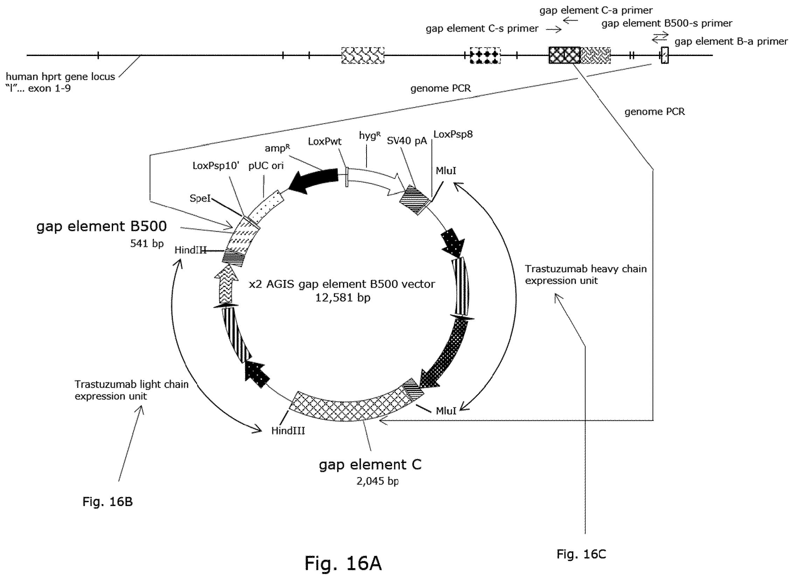

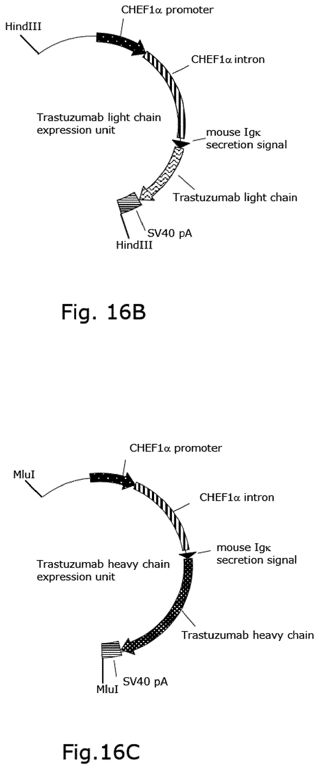

The gap element C was inserted between the Mlul and Hindlll sites of the x2 basic vector prepared in Step (1-4). Then, the gap element B500 was inserted between the Hindlll and Spel sites, and subsequently the fragment of the trastuzumab heavy chain expression unit of Step (1-1) was inserted in the Mlul site. Finally, the fragment of the trastuzumab light chain expression unit of Step (1-2) was inserted in the Hindlll site to prepare the x2 AGIS gap element B500 vector shown in Fig. 16A.

Next, the x2 AGIS gap element B500 vector was accumulatively integrated into the vector 1-integrated cell line as follows to prepare the cells.

The x2 AGIS gap element B500 vector was integrated into the vector 1-integrated cell line, prepared in Step (2-4), in the same manner as in Step (2-3) to obtain a positive clone.

Genomic DNA of the positive clone obtained was extracted with MAG Extractor. The genomic DNA extracted was analyzed by genomic PCR using a primer set of primer 9/primer 10 and a primer set of primer 11/primer 12.

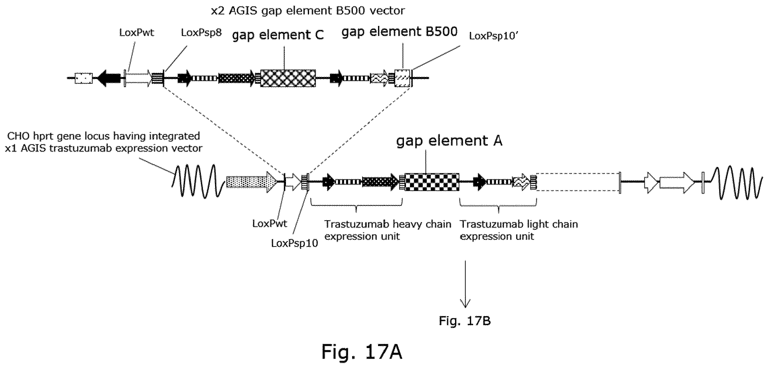

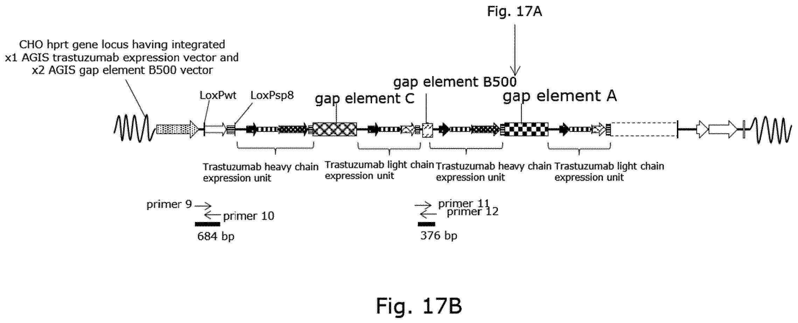

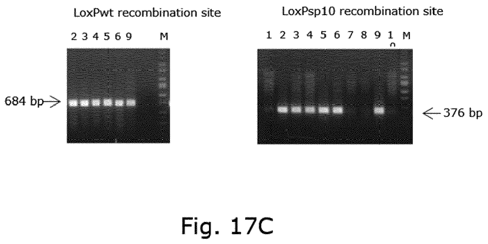

As shown in Fig. 17B, in the case of a correctly accumulatively integrated clone, a PCR product of 684 bp is amplified in the vicinity of LoxPwt recombination site. Further, a PCR product of 376 bp is amplified in the vicinity of LoxPsp10 recombination site. The result of analysis was as shown in Fig. 17C. When analysis of the vicinity of LoxPsp10 recombination site was performed, in 6 clones out of 10 clones, the amplification product of 376 bp was amplified. When the 6 clones were analyzed in the vicinity of LoxPwt recombination site, in 6 clones out of the 6 clones, the amplification product of 684 bp was amplified.

Clone No. 2 was selected and, in the same manner as in Evaluation 1, the copy numbers of the heavy chain and light chain of trastuzumab in the genomic DNA was quantified by real-time PCR.

As a result, the copy number of trastuzumab heavy chain was 1.9±0.1 and the copy number of light chain was 2.0±0.2, which were approximately in agreement with 2, the number of genes introduced. Thus, clone No. 2 was used in the following evaluation as vector 1- and x2 AGIS gap element B500 vector-introduced cell line (hereinafter, this cell is called "Example 3").

### Evaluation: Evaluation of Vector-Integrated CHO Cell Line, the Vector Having One Gap Element of 0.5 kbp (541 bp)

### Evaluation 4: Analysis of Expression Levels of Trastuzumab Heavy Chain and Light Chain in Cells of Example 3

The cells of Example 3 were maintained by subculture in the same manner as in Evaluation 2, and the production amount of trastuzumab in the cell line was timely measured.

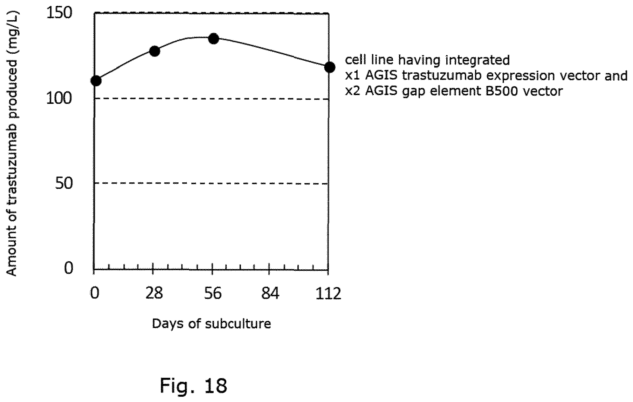

The expression level of trastuzumab in batch culture of Example 3 was 110 mg/L immediately after establishment. The expression level was 119 mg/L after 112 days of subculture, and thus the expression level was stably maintained (Fig. 18).

### Example 4: Example of Cell Having One Gap Element of 0.5 kbp (#2) Example of Cell in which Gap Element C of Example 1 is Changed to Chain Length of 0.5 kbp

A "x2 AGIS Gap Element C500 Vector" having one gap element of 493 bp was prepared as follows.

Using genomic DNA of HEK293 as a template, a gap element C500 (SEQ ID NO: 69) of 493 bp was PCR amplified with a gap element C500-s/gap element C-a primer set, and the length of amplification product was confirmed by agarose gel electrophoresis. The recognition sequence (underlined part) for restriction endonuclease was attached to the 5' terminal of the sense primer.
gap element C500-s primer:
5'-AAGCTTTCATCTCTCAACAACTCTGG-3' (SEQ ID NO: 72)

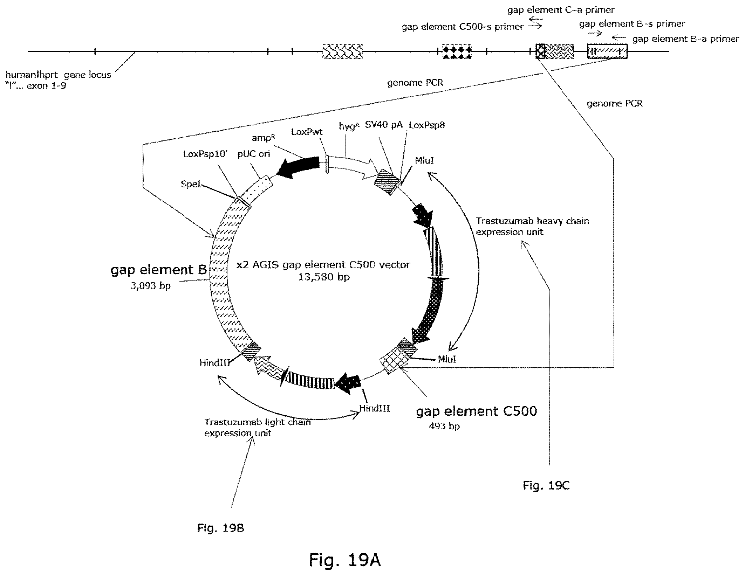

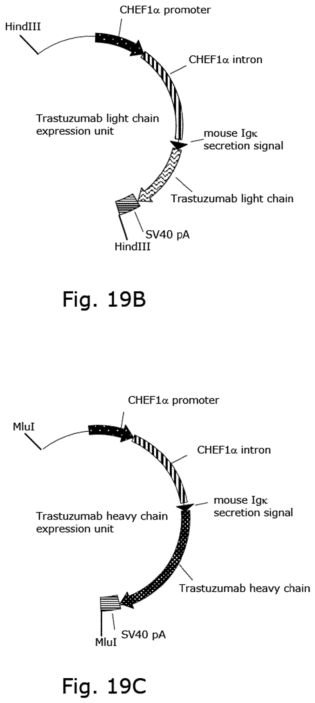

The gap element C500 was inserted between the Mlul and Hindlll sites of the x2 basic vector prepared in Step (1-4). Then, the gap element B was inserted between the Hindlll and Spel sites, and subsequently the fragment of the trastuzumab heavy chain expression unit of Step (1-1) was inserted in the Mlul site. Finally, the fragment of the trastuzumab light chain expression unit of Step (1-2) was inserted in the Hindlll site to prepare the x2 AGIS gap element C500 vector shown in Fig. 19A.

Next, the x2 AGIS gap element C500 vector was accumulatively integrated into the vector 1-integrated cell line as follows to prepare the cells.

The x2 AGIS gap element C500 vector was integrated into the vector 1-integrated cell line, prepared in Step (2-4), in the same manner as in Step (2-3) to obtain a positive clone.

Genomic DNA of the positive clone obtained was extracted with MAG Extractor. The genomic DNA extracted was analyzed by genomic PCR using a primer set of primer 9/primer 10 and a primer set of primer 11/primer 12.

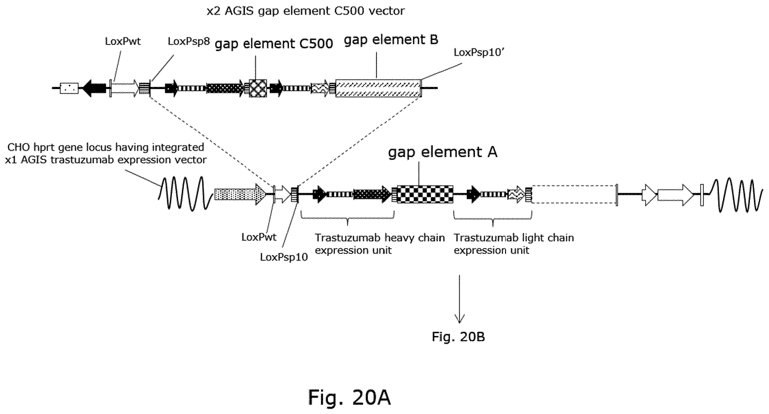

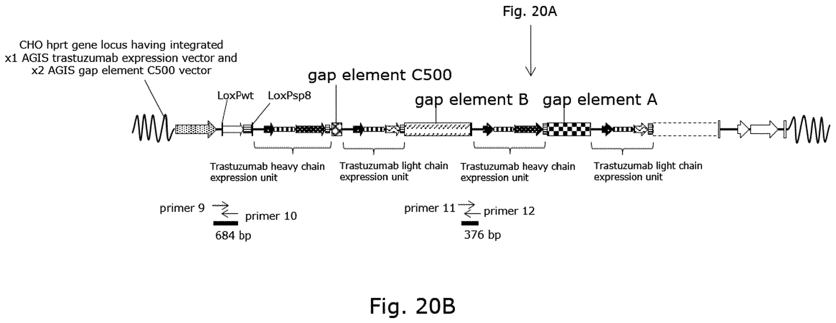

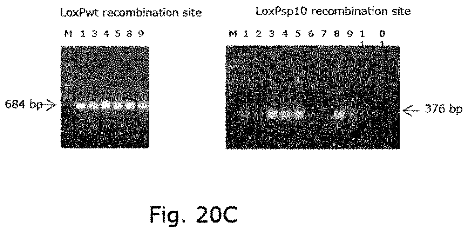

As shown in Fig. 20B, in the case of a correct accumulatively integrated clone, a PCR product of 684 bp is amplified in the vicinity of LoxPwt recombination site. Further, a PCR product of 376 bp is amplified in the vicinity of LoxPsp10 recombination site. The result of analysis was as shown in Fig. 20C. When analysis of the vicinity of LoxPsp10 recombination site was performed, in 6 clones out of 11 clones, the amplification product of 376 bp was amplified. When the 6 clones were analyzed in the vicinity of LoxPwt recombination site, in 6 clones out of the 6 clones, the amplification product of 684 bp was amplified.

Clone No. 5 was selected and, in the same manner as in Evaluation 1, the copy numbers of the heavy chain and light chain of trastuzumab in the genomic DNA was quantified by real-time PCR.

As a result, the copy number of trastuzumab heavy chain was 2.0±0.4 and the copy number of trastuzumab light chain was 1.8±0.5, which were approximately in agreement with 2, the number of genes introduced. Thus, clone No. 5 was used in the following evaluation as vector 1- and x2 AGIS gap element C500 vector-integrated cell line (hereinafter, this cell is called "Example 4").

### Evaluation: Evaluation of Vector-Integrated CHO Cell Line, the Vector Having One Gap Element of 0.5 kbp (493 bp)

### Evaluation 5: Analysis of Expression Levels of Trastuzumab Heavy Chain and Light Chain in Cells of Example 4

The cells of Example 4 were maintained by subculture in the same manner as in Evaluation 2, and the production amount of trastuzumab in the cell line was timely measured.

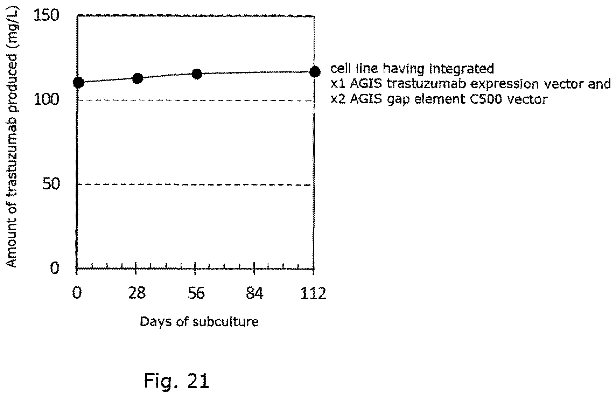

The expression level of trastuzumab in batch culture of Example 4 was 111 mg/L immediately after establishment. The expression level was 118 mg/L after 102 days of subculture, and thus the expression level was stably maintained (Fig. 21).

### Example 5: Example of Cell in which Gap Elements A. B, and C of Example 1 are All Changed to Chain Length of 0.5 kbp

A "x1 AGIS Gap Element A500 Vector" having one gap element of 502 bp was prepared as follows.

Using genomic DNA of HEK293 as a template, a gap element A500 (SEQ ID NO: 70) of 502 bp was PCR amplified with a primer set of gap element A500-s/gap element A-a, and the length of amplification product was confirmed by agarose gel electrophoresis. To the 5' terminal of the sense primer was attached the recognition sequence (underlined part) for restriction endonuclease HindIII.
gap element A500-s primer:
5'- AAGCTTGGGGGTATTTTTGTGTGCAG-3' (SEQ ID NO: 71)

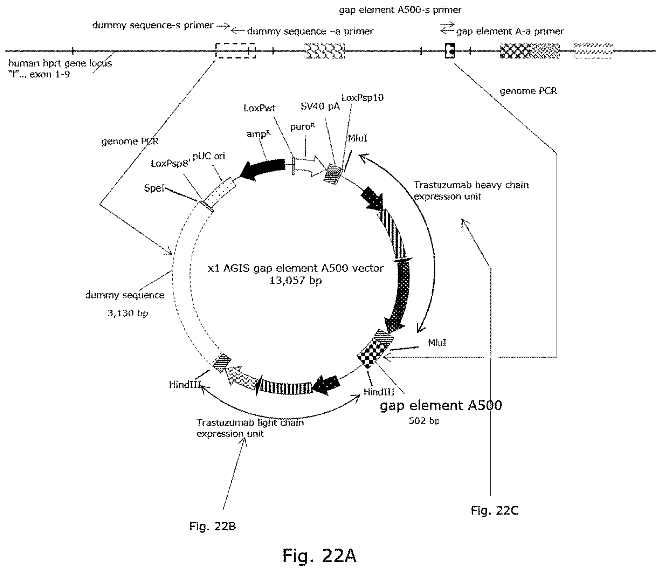

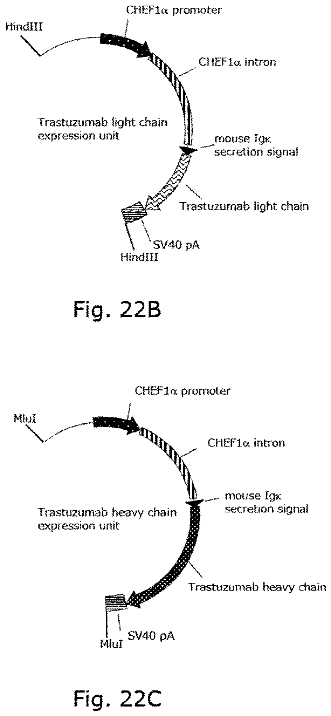

The gap element A500 was inserted between the Mlul and Hindlll sites of the x1 basic vector prepared in Step (1-3). Then, a dummy sequence was inserted between the Hindlll and Spel sites, and subsequently the fragment of the trastuzumab heavy chain expression unit of Step (1-1) was inserted in the Mlul site. Finally, the fragment of the trastuzumab light chain expression unit of Step (1-2) was inserted in the Hindlll site to prepare the x1 AGIS gap element A500 vector shown in Fig. 22A.

Next, a "x2 AGIS gap element C500-B500 vector" having two gap elements of 493 bp and 541 bp, respectively, was prepared as follows.

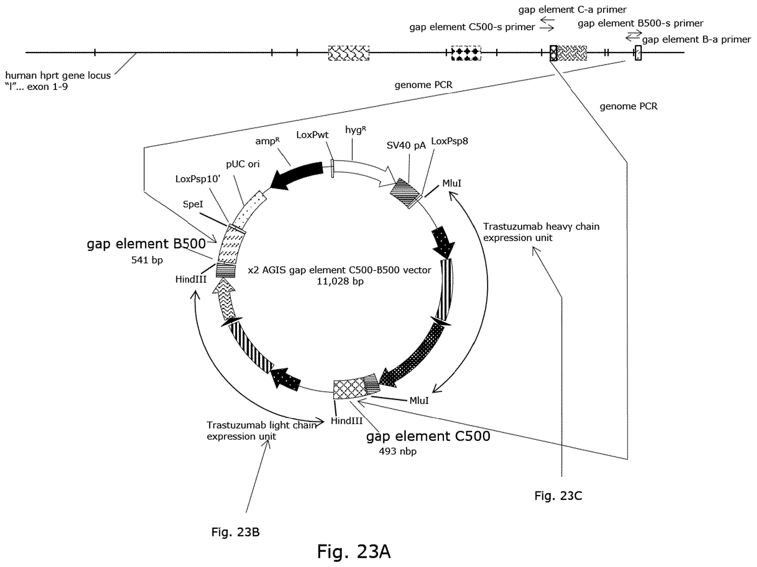

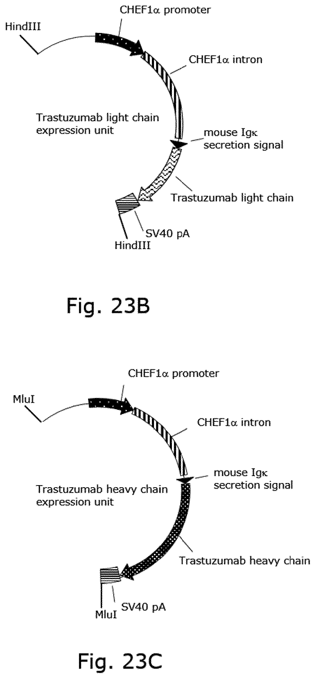

The gap element C500 of Step (5-1) was inserted between the Mlul and Hindlll sites of the x2 basic vector prepared in Step (1-4). Then, the gap element B500 of Step (4-1) was inserted between the Hindlll and Spel sites, and subsequently the fragment of the trastuzumab heavy chain expression unit of Step (1-1) was inserted in the Mlul site. Finally, the fragment of the trastuzumab light chain expression unit of Step (1-2) was inserted in the Hindlll site to prepare the x2 AGIS gap element C500-B500 vector shown in Fig. 23A.

Next, the x1 AGIS gap element A500 vector was accumulatively integrated into the CHO hprt AGIS origin vector-integrated cell line as follows.

Into the CHO hprt AGIS origin vector-integrated cell line, prepared in process (2-3), 2 µg of x1 AGIS gap element A500 vector and 0.2 µg of Cre expression plasmid were introduced by voltage application in the same manner as in Step (2-4), and the cell line was seeded on a 96-well plate.

To exchange culture medium after 2 days and 6 days from seeding, and for cell expansion, there was used AMEM medium+puromycin (final concentration: 12 µg/mL).

The cells which showed proliferation in the well were collected to obtain x1 AGIS gap element A500 vector-integrated cells.

Then, into the x1 AGIS gap element A500 vector-integrated cell line, the x2 AGIS gap element C500-B500 vector was accumulatively integrated as follows to prepare the cells of Example 5.

Into the x1 AGIS gap element A500 vector-integrated cell line prepared in Step (6-3), the x2 AGIS gap element C500-B500 vector was integrated in the same manner as in Step (2-3) to obtain a positive clone.

Genomic DNA of the positive clone obtained was extracted with MAG Extractor. The genomic DNA extracted was analyzed by genomic PCR using a primer set of primer 7/primer 8, a primer set of primer 9/primer 10, and a primer set of primer 11/primer 12.

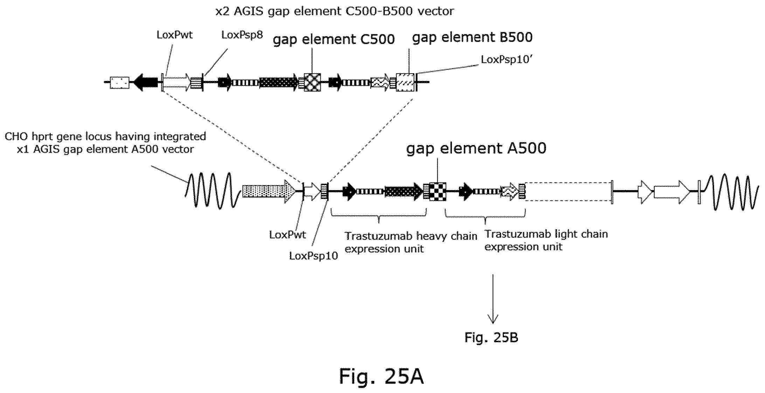

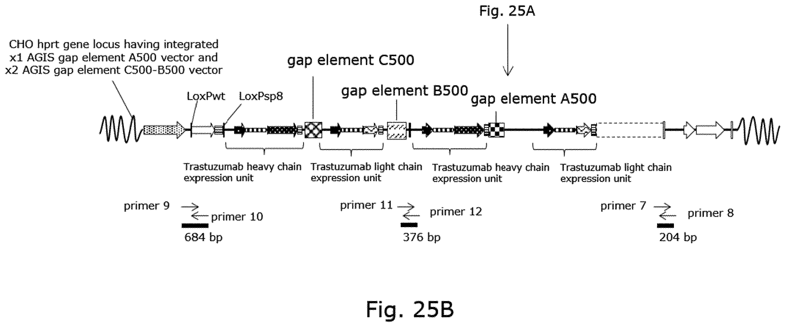

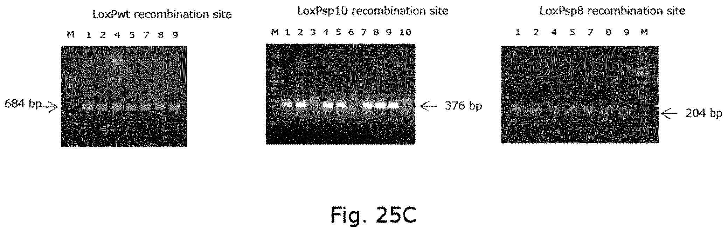

As shown in Fig. 25B, in the case of a correct accumulatively integrated clone, a PCR product of 684 bp is amplified in the vicinity of LoxPwt recombination site. Further, a PCR product of 376 bp is amplified in the vicinity of LoxPsp10 recombination site. Furthermore, a PCR product of 204 bp is amplified in the vicinity of LoxPsp8 recombination site. The result of analysis was as shown in Fig. 25C. When analysis of the vicinity of LoxPsp10 was performed, in 7 clones out of 10 clones, the product of 376 bp was amplified. When the 7 clones were analyzed in the vicinity of LoxPwt recombination site, in 7 clones out of the 7 clones, the amplification product of 684 bp was amplified. Also, when the 7 clones were analyzed in the vicinity of LoxPsp8 recombination site, in 7 clones out of the 7 clones, the amplification product of 204 bp was amplified.

Clone No. 2 was selected and, in the same manner as in Evaluation 1, the copy numbers of the heavy chain and light chain of trastuzumab in the genomic DNA were quantified by real-time PCR.

As a result, the copy number of trastuzumab heavy chain was 1.8±0.1 and the copy number of light chain was 2.0±0.3, which were approximately in agreement with 2, the number of genes introduced. Thus, clone No. 2 was used in the following evaluation as x1 AGIS gap element A500 vector and x2 AGIS gap element C500-B500 vector -introduced cell line (hereinafter, this cell is called "Example 5").

### Evaluation: Evaluation of Vector-Integrated CHO Cell Line in which All of 3 Gap Elements are 0.5 kbp

### Evaluation 6: Analysis of Expression Levels of Trastuzumab Heavy Chain and Light Chain in Cells of Example 5

The cells of Example 5 were maintained by subculture in the same manner as in Evaluation 2, and the production amount of trastuzumab in the cell line was timely measured.

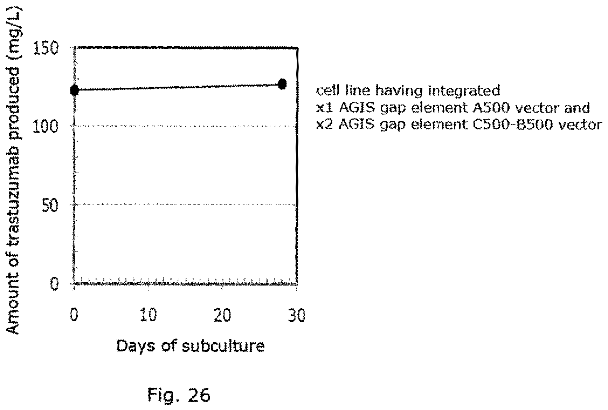

The expression level of trastuzumab in batch culture of Example 5 was 123 mg/L immediately after establishment. The expression level was 127 mg/L after 28 days of subculture, and thus was stably maintained (Fig. 26).

## Claims

1. A mammalian cell comprising multiple expression units which are integrated therein and comprise exogenous genes of interest which are integrated therein, wherein the number of said multiple expression units is 3 or more; and further comprising exogenous gap elements among said multiple expression units wherein said gap elements have chain lengths of 0.5 kbp or more and 20 kbp or less, and said gap elements are mutually different sequences, and homology between said mutually different gap elements is 95% or less.

2. The cell according to claim 1, wherein the chain length of said gap elements is in the range from 1 kbp or more to 20kbp or less.

3. The cell according to claim 1, wherein one or more of said gap elements have chain lengths of 1 kbp or more and 20 kbp or less.

4. The cell according to any one of claims 1 to 3, wherein multiple kinds of said genes of interest are integrated.

5. The cell according to any one of claims 1 to 4, wherein said multiple expression units and said gap elements are introduced into said animal cell.

6. The cell according to claim 5, wherein said multiple expression units and said gap elements are introduced into a chromosome of said animal cell.

7. The cell according to claim 5 or 6, wherein a site of said chromosome of said animal cell where said multiple expression units and said gap elements are introduced is a stable expression gene locus.

8. The cell according to claim 7, wherein said gene locus is a hypoxanthine-guanine phosphoribosyltransferase (hprt) gene locus.

9. The cell according to any one of claims 1 to 8, wherein homology between said gap elements adjacent to each other is either of 55% or less or 50% or less.

10. The cell according to any one of claims 1 to 8, wherein homology between said mutually different gap elements is either of 55% or less or 50% or less.

11. The cell according to any one of claims 1 to 10, wherein said mammalian cell is a CHO cell.

12. The cell according to any one of claims 1 to 11, wherein said gene of interest is a gene which encodes a protein.

13. The cell according to claim 12, wherein said protein is an antibody.

14. The cell according to claim 12 or 13, wherein said protein is a monoclonal antibody.

15. A method for producing a substance of interest, comprising a step of culturing a cell according to any one of claims 1 to 14.

16. A method for producing the cell according to any one of claims 1 to 14, wherein said multiple expression units and said gap elements are introduced by using accumulative gene integration system (AGIS).

## Patentansprüche

1. Säugerzelle, umfassend mehrere darin integrierte Expressionseinheiten mit darin integrierten exogenen Genen von Interesse, wobei die Anzahl der mehreren Expressionseinheiten 3 oder mehr beträgt; und ferner umfassend exogene Abstandselemente unter den mehreren Expressionseinheiten, wobei die Abstandselemente Kettenlängen von 0,5 kbp oder mehr und 20 kbp oder weniger aufweisen, und die Abstandselemente voneinander verschiedene Sequenzen sind, und die Homologie zwischen den voneinander verschiedenen Abstandselementen 95 % oder weniger beträgt.

2. Zelle nach Anspruch 1, wobei die Kettenlänge der Abstandselemente im Bereich von 1 kbp oder mehr bis 20 kbp oder weniger liegt.

3. Zelle nach Anspruch 1, wobei eines oder mehrere der Abstandselemente Kettenlängen von 1 kbp oder mehr und 20 kbp oder weniger aufweist.

4. Zelle nach einem der Ansprüche 1 bis 3, wobei mehrere Arten der Gene von Interesse integriert sind.

5. Zelle nach einem der Ansprüche 1 bis 4, wobei die mehreren Expressionseinheiten und die Abstandselemente in die Tierzelle eingeführt sind.

6. Zelle nach Anspruch 5, wobei die mehreren Expressionseinheiten und die Abstandselemente in ein Chromosom der Tierzelle eingeführt sind.

7. Zelle nach Anspruch 5 oder 6, wobei eine Stelle des Chromosoms der Tierzelle, an welcher die mehreren Expressionseinheiten und die Abstandselemente eingeführt sind, ein Genort mit stabiler Expression ist.

8. Zelle nach Anspruch 7, wobei der Genort ein Hypoxanthin-Guanin Phosphoribosyltransferase (hprt) Genort ist.

9. Zelle nach einem der Ansprüche 1 bis 8, wobei die Homologie zwischen den aneinandergrenzenden Abstandselementen entweder 55 % oder weniger oder 50 % oder weniger beträgt.

10. Zelle nach einem der Ansprüche 1 bis 8, wobei die Homologie zwischen den voneinander verschiedenen Abstandselementen entweder 55 % oder weniger oder 50 % oder weniger beträgt.

11. Zelle nach einem der Ansprüche 1 bis 10, wobei die Säugerzelle eine CHO-Zelle ist.

12. Zelle nach einem der Ansprüche 1 bis 11, wobei das Gen von Interesse ein Gen ist, das für ein Protein kodiert.

13. Zelle nach Anspruch 12, wobei das Protein ein Antikörper ist.

14. Zelle nach Anspruch 12 oder 13, wobei das Protein ein monoklonaler Antikörper ist.

15. Verfahren zur Herstellung einer Verbindung von Interesse, umfassend einen Schritt zur Kultivierung einer Zelle nach einem der Ansprüche 1 bis 14.

16. Verfahren zur Herstellung der Zelle nach einem der Ansprüche 1 bis 14, wobei die mehreren Expressionseinheiten und die Abstandselemente mit Hilfe des akkumulativen Genintegrationssystems (AGIS) eingeführt werden.

## Revendications

1. Une cellule de mammifère comprenant des unités d'expression multiples qui sont intégrées à elle et qui comprennent des gènes d'intérêt exogènes qui sont intégrés à elles, le nombre desdites unités d'expression multiples étant de 3 ou plus ; et comprenant en outre des éléments d'écart exogènes parmi lesdites unités d'expression multiples, lesdits éléments d'écart ayant des longueurs de chaîne de 0,5 kpb ou plus et de 20 kpb ou moins, et lesdits éléments d'écart étant des séquences mutuellement différentes, et l'homologie entre lesdits éléments d'écart mutuellement différents étant de 95 % ou moins.

2. La cellule selon la revendication 1, dans laquelle la longueur de chaîne desdits éléments d'écart est dans une gamme allant de 1 kpb ou plus à 20 kpb ou moins.

3. La cellule selon la revendication 1, dans laquelle un ou plusieurs desdits éléments d'écart ont des longueurs de chaîne de 1 kpb ou plus et de 20 kpb ou moins.

4. La cellule selon l'une quelconque des revendications 1 à 3, dans laquelle plusieurs types desdits gènes d'intérêt sont intégrés.

5. La cellule selon l'une quelconque des revendications 1 à 4, dans laquelle lesdites unités d'expression multiples et lesdits éléments d'écart sont introduits dans ladite cellule animale.

6. La cellule selon la revendication 5, dans laquelle lesdites unités d'expression multiples et lesdits éléments d'écart sont introduits dans un chromosome de ladite cellule animale.

7. La cellule selon la revendication 5 ou la revendication 6, dans laquelle un site dudit chromosome de ladite cellule animale dans lequel lesdites unités d'expression multiples et lesdits éléments d'écart sont introduits est un locus de gène d'expression stable.

8. La cellule selon la revendication 7, dans laquelle ledit locus du gène est un locus du gène de l'hypoxanthine-guanine phosphoribosyltransférase (hprt).

9. La cellule selon l'une quelconque des revendications 1 à 8, dans laquelle l'homologie entre lesdits éléments d'écart adjacents les uns aux autres est de 55 % ou moins ou de 50 % ou moins.

10. La cellule selon l'une quelconque des revendications 1 à 8, dans laquelle l'homologie entre lesdits éléments d'écart mutuellement différents est de 55 % ou moins ou de 50 % ou moins.

11. La cellule selon l'une quelconque des revendications 1 à 10, dans laquelle ladite cellule de mammifère est une cellule CHO.

12. La cellule selon l'une quelconque des revendications 1 à 11, dans laquelle ledit gène d'intérêt est un gène qui code pour une protéine.

13. La cellule selon la revendication 12, dans laquelle ladite protéine est un anticorps.

14. La cellule selon la revendication 12 ou la revendication 13, dans laquelle ladite protéine est un anticorps monoclonal.

15. Un procédé de production d'une substance d'intérêt, comprenant une étape de culture d'une cellule selon l'une quelconque des revendications 1 à 14.

16. Un procédé de production de la cellule selon l'une quelconque des revendications 1 à 14, dans lequel lesdites unités d'expression multiples et lesdits éléments d'écart sont introduits en utilisant un système d'intégration de gènes accumulés (AGIS).


### SEQUENCE LISTING

<110> TOTO LTD. KYUSHU UNIVERSITY, NATIONAL UNIVERSITY CORPORATION
<120> Recombinant mammalian cells and method for producing substance of interest
<130> P16067EP00
<150> JP 2017-071936
   <151> 2017-03-31
<160> 71
<170> PatentIn version 3.5
<210> 1
   <211> 21
   <212> PRT
<221> PEPTIDE
<400> 1
   Met Glu Thr Asp Thr Leu Leu Leu Trp Val Leu Leu Leu Trp Val Pro
   1               5                   10                  15
   Gly Ser Thr Gly Asp
               20
<210> 2
   <211> 26
   <212> DNA
   <213> Artificial Sequence
<400> 2
   ctcgagctcg atgagtttgg acaaac 26
<210> 3
   <211> 49
   <212> DNA
   <213> Artificial Sequence
<400> 3
   atcgatgtcg acaagctttc tagaacgcgt taccacattt gtagaggtt 49
<210> 4
   <211> 49
   <212> DNA
   <213> Artificial Sequence
<400> 4
   atcgatgtcg acaagctttc tagaacgcgt ccacacaatc agaaccaca 49
<210> 5
   <211> 26
   <212> DNA
   <213> Artificial Sequence
<400> 5
   gaattcgcgg tggttttcac aacacc 26
<210> 6
   <211> 26
   <212> DNA
   <213> Artificial Sequence
<400> 6
   atcgatgctt acatggcgat agctag 26
<210> 7
   <211> 26
   <212> DNA
   <213> Artificial Sequence
<400> 7
   atcgatgaat caggggataa cgcagg 26
<210> 8
   <211> 60
   <212> DNA
   <213> Artificial Sequence
<400> 8
   actagtctac cgttcgtata aagtatccta tacgaagtta ttagcttcag tgagcaaaag 60
<210> 9
   <211> 26
   <212> DNA
   <213> Artificial Sequence
<400> 9
   ggatcctcgc gtaggtggca cttttc 26
<210> 10
   <211> 61
   <212> DNA
   <213> Artificial Sequence
<400> 10
   ggatccataa cttcgtatag catacattat acgaagttat ccaccgagtc aagcccacgg 60
   t 61
<210> 11
   <211> 72
   <212> DNA
   <213> Artificial Sequence
<400> 11
   actagtaagc ttacgcgtta ccgttcgtat aattatggtt atacgaagtt atacgatcat 60
   aatcagccat ac 72
<210> 12
   <211> 26
   <212> DNA
   <213> Artificial Sequence
<400> 12
   aagcttggca acatagcgag acttcg 26
<210> 13
   <211> 26
   <212> DNA
   <213> Artificial Sequence
<400> 13
   actagtgtgt acagaaggct gaaagg 26
<210> 14
   <211> 2051
   <212> DNA
   <213> Artificial Sequence
<400> 14
   tgaaagtgtg agttccggag cctgacaact gattcaaagc atggcttagt acttcctaac      60
   tctgaccttg ggcaagttac ttaacctctc tgtgtcccat atgtgattag ggtgaggttg     120
   ataatagcag ccatagagtt aagaggatta agtgctataa tgcaagtaga gctcttacaa     180
   cagtttctgg taaatcactc aataaattca gacatactat tattttaaga aatctcaaag     240
   agttttcttg taccttaaaa ttctcctagt gtgaaccatt ggttttggta tattgtgctt     300
   ccatgtagtt taatatcaag atgtttttag atttcccttt taatttattt gttgacccat     360
   tggttgttca ggagcatgct gtttacctga aaataatgga gatattaagg tatttgaata     420
   tttatcttct agtacattga aaaacttttt gagagtaacc aataataaat gatggaatgc     480
   tactgctttt tttttttgaa gctgccagtt attgtttact tacactatgc caaatataaa     540
   ggcattaatc tcataaaagt ttcacaacaa tcctgtgagg gagacgatat ccccatttta     600
   caaatcagga aattaagact taataaggtt aaaagacttg cccaaagtca cagaaccagt     660
   aagtggtaga gcttgaattt gaatacagac ctgactctaa agctcttttc tttctttaga     720
   ttttagtgtt cattgcttac ttgaatgagt atctataaga aaactttaac atgtaaaact     780
   tctgtgaaat tatcttgtcc catatcaggg tcatgtcaaa ctaatgtcct cctcagcatc     840
   tttggaaaac ttcagaggag aaatgagctt tgcccctcct gttcatttca tataccactg     900
   ttagacctgt ccttcccttt cagcatgctt tgtccatatt tagaagctgt tgaagccatt     960
   acttgtctgg tcagttttta gtgctggaat ggacctagcc ttttaggcct tctgagattt    1020
   agtttgatct cgtctttccc acctaatggc tctgttctac tacatagatt tgatctgaaa    1080
   cagttctctg tttctaaaat aactttcttt tcatgatagt cacagtaaag tacatttatt    1140
   atggaaaaat caataagtat aacgagtgaa agttatttct tggtggtaag attatgggat 1200
   tatttgaact ttctgtttca ttgtatttta tttatttatt tatttttgtg atggagtctc 1260
   actctgctgc ccaggctgga gtgcagtagt acgatcttgg ctcactgcaa cctccccttc 1320
   ccagttcaag tgattctcct gcctcagact cccaagtagc tgggattaca ggcgcacgcc 1380
   accatgcctg gctaattttt ttatctttag tagagacagg gtttcaccat gttgaccagg 1440
   ctgatctcca actcctgatc tcaggtatcc acctgcctca gcctcccaaa gtaccgggat 1500
   tacgggtgtg agccaccctg cctggcctca ttttgtcttt tgggggtatt tttgtgtgca 1560
   gatatatatg tatataaata tttttccctc ttttccccag ttagtatttg agcagatgaa 1620
   ctttggaccc gaatacctgt attcaagtct ctaataccac ttcttggcta ttttcatttt 1680
   atcaaatggc ctcttatcct cgtttttctc atttattaag tagagatgta actacttgat 1740
   ataattcaaa aactcaataa tggcattctt ttgtttttta gactctagtg tctgtactcg 1800
   ttgtaccatg ctgggattca tttgaacaat tgcatggctt ttttagtgta ttattaaatt 1860
   tgcagtttac ttagaattta ctgggacctc atacaaatgg gaaaaaaaca taactgtgtt 1920
   actcatttgc tgtgtgcctt tggattgacc ctattttttg tattcatttt ctccccatgt 1980
   cctgagttcc actttgaata aaaagtaatt tttttcctgc ctgtaaaata ggctaccaat 2040
   aggctgcagt t 2051
<210> 15
   <211> 26
   <212> DNA
   <213> Artificial Sequence
<400> 15
   acgcgttgaa agtgtgagtt ccggag 26
<210> 16
   <211> 52
   <212> DNA
   <213> Artificial Sequence
<400> 16
   aagcttaact gcagcctatt ggtagcacgc gttgaaagtg tgagttccgg ag 52
<210> 17
   <211> 61
   <212> DNA
   <213> Artificial Sequence
<400> 17
   actagtctac cgttcgtata accataatta tacgaagtta ttagcttcat gtgagcaaaa 60
   g 61
<210> 18
   <211> 26
   <212> DNA
   <213> Artificial Sequence
<400> 18
   ggatcctcgc gtaggtggca cttttc 26
<210> 19
   <211> 61
   <212> DNA
   <213> Artificial Sequence
<400> 19
   ggatccataa cttcgtatag catacattat acgaagttat ccaaaaagct gaactcaccg 60
   c 61
<210> 20
   <211> 72
   <212> DNA
   <213> Artificial Sequence
<400> 20
   actagtaagc ttacgcgtta ccgttcgtat aggatacttt atacgaagtt attagctcac 60
   tcattaggca cc 72
<210> 21
   <211> 26
   <212> DNA
   <213> Artificial Sequence
<400> 21
   acgcgtcgtt ttctcactgg ctgctg 26
<210> 22
   <211> 26
   <212> DNA
   <213> Artificial Sequence
<400> 22
   aagcttactc tcatagtacc tgtccc 26
<210> 23
   <211> 3093
   <212> DNA
   <213> Artificial Sequence
<400> 23
   gctcattgac actctgtacc cattaatcct ccttattagc tccccttcaa tggacacatg      60
   ggtagtcagg gtgcaggtct cagaactgtc cttcaggttc caggtgatca accaagtgcc     120
   ttgtctgtag tgtcaactca ttgctgcccc ttcctagtaa tccccataat ttagctctcc     180
   atttcatagt ctttccttgg gtgtgttaaa agtgaccatg gtacactcag cacggatgaa     240
   atgaaacagt gtttagaaac gtcagtcttc tcttttgtaa tgccctgtag tctctctgta     300
   tgttatatgt cacattttgt aattaacagc ttgctggtga aaaggacccc acgaagtgtt      360
   ggatataagc cagactgtaa gtgaattact ttttttgtca atcatttaac catctttaac      420
   ctaaaagagt tttatgtgaa atggcttata attgcttaga gaatatttgt agagaggcac      480
   atttgccagt attagattta aaagtgatgt tttctttatc taaatgatga attatgattc      540
   tttttagttg ttggatttga aattccagac aagtttgttg taggatatgc ccttgactat      600
   aatgaatact tcagggattt gaatgtaagt aattgcttct ttttctcact catttttcaa      660
   aacacgcata aaaatttagg aaagagaatt gttttctcct tccagcacct cataatttga      720
   acagactgat ggttcccatt agtcacataa agctgtagtc tagtacagac gtccttagaa      780
   ctggaacctg gccaggctag ggtgacactt cttgttggct gaaatagttg aacagcttta      840
   atatacaata attgttgcat tattatttca gatgataaat gtggtcataa gtaagaaata      900
   aatgatcgag tttagtcttt taattcactg tcctttgaat acctgcctct tactctggag      960
   gcagaagtcc catggatgtg tttatgaaca tggttgagga agatttagga agactgcaac     1020
   agtacactac ctaaagcagg ttttttactc catctttttt tgccacgtac actggcctcc     1080
   cactttgata tgcttgaaat tatctccttg atttgtcttt caaaactaca tattgaggct     1140
   ggttgcggtg gctcacacct gtaatcctag cactttggga ggccaagccg gacagatcac     1200
   ttgaggtcag gagttcgaga ccagcctggc aaacatgatg aaaccccacc tttactaaaa     1260
   atacaaaaat tagccaggcg tagtggtgtg tgcctgtaac ccagctacct gggaggctga     1320
   ggcaggagaa tcactggaac ccgggaggca gaggctacag tgagccaaca tcacgccact     1380
   gcactccagc ctgggtgaca gagcaagact ctgtctcaaa acaaaacaaa aaacaaaaaa     1440
   ctacgtatta agacaagaaa cagactgggc gcggtggctc acgcctgtaa tcccagcact     1500
   ttgggaggct gaggcgggcg gatcacaagg tcaggagatc gagaccatcc tggctaacac     1560
   ggtgaaaccc cgtctctact aaaaaataga aaaaattagc tggggtggtg gcgggcgcct     1620
   atagtctcag ctactcggga ggctgaggca ggagaatggc gtgaacccgg gaggcagagc     1680
   ttgcagtgag cagagatcgt gccactgcac tccagtctgg gtgacagagc aagactccgt     1740
   ctcaaaaaaa aaaacaaaaa caagaaacaa attaaactaa tgtgatagac tactgctttg     1800
   ttttcaaaag atacactccc caaaagttac tgatctaaaa tacagtagta ctatctctgt     1860
   ttagtaagaa ccctgacaac taatagtgtt cttatatgta aaatgctatt cttgcctttc     1920
   atttcagaat atacttttta aatgtgaatt tctggatttt tttttatagc atgtttgtgt    1980
   cattagtgaa actggaaaag caaaatacaa agcctaagat gagagttcaa gttgagtttg     2040
   gaaacatctg gagtcctatt gacatcgcca gtaaaattat caatgttcta gttctgtggc     2100
   catctgctta gtagagcttt ttgcatgtat cttctaagaa ttttatctgt tttgtacttt     2160
   agaaatgtca gttgctgcat tcctaaactg tttatttgca ctatgagcct atagactatc 2220
   agttcccttt gggcggattg ttgtttaact tgtaaatgaa aaaattctct taaaccacag 2280
   cactattgag tgaaacattg aactcatatc tgtaagaaat aaagagaaga tatattagtt 2340
   ttttaattgg tattttaatt tttatatatg caggaaagaa tagaagtgat tgaatattgt 2400
   taattatacc accgtgtgtt agaaaagtaa gaagcagtca attttcacat caaagacagc 2460
   atctaagaag ttttgttctg tcctggaatt attttagtag tgtttcagta atgttgactg 2520
   tattttccaa cttgttcaaa ttattaccag tgaatctttg tcagcagttc ccttttaaat 2580
   gcaaatcaat aaattcccaa aaatttaact gctttatgaa ttcaatttaa aaatccttaa 2640
   aataagtcct gtctctttaa aaaaacctat gcatagttat catttctcta caaattaacc 2700
   tagtttagtt ttctgttggt tccattttcc ttgtttgtta agttttagta gctagtttaa 2760
   ttgtaatctc aatgattatg tggtagaatg ggttggcgga gctacaaaaa ttcctagcta 2820
   cttcagagac attaaatttc agacacatgg tacactttat attacatttt actatgctaa 2880
   aataacacgg ctttcttttg gaattctgtt cagtttttca gattgtaatc tcagctacat 2940
   ctcaacagat tgttctcaga tatgtcctat taccttcttt gtgtagatag tgctttattg 3000
   actaagaaca atgacaacaa caccttttgt tttctgggaa taggagaaaa gttttaagcc 3060
   aaaactctta attgcttatc tgctccacgt gag 3093
<210> 24
   <211> 26
   <212> DNA
   <213> Artificial Sequence
<400> 24
   aagcttgctc attgacactc tgtacc 26
<210> 25
   <211> 26
   <212> DNA
   <213> Artificial Sequence
<400> 25
   actagtctca cgtggagcag ataagc 26
<210> 26
   <211> 2045
   <212> DNA
   <213> Artificial Sequence
<400> 26
   cgttttctca ctggctgctg gccaggggtt gctcccagat atttaaaggc tcatgcccta      60
   gcccatgaca gtctcacaac atggcagctg acttcttcaa aaccagcagg agaatcttgc     120
   tctagtctac cacataacct aatcacagga gcggctatcc cgttattttc acagatcctg     180
   gtcacattca aggggaggga acccttctgt gtgtgtacac caggaggcag gaattttttt      240
   tttctttttc ttttttgtta aaaagtctta aagtctttta tccctaaagg aggcaggaat      300
   tttgagagcc atcagaattc tgcctaccac agcccagaaa tctgcatttt tcacaagtct      360
   ccagccatga tgtttctgat ggctcacact gctttattcc atttttaaag agtattttta      420
   ttgaaaagca ttagggttat ggtttaaaaa atattttccc taacaaagat gggtttgttt      480
   agagtcctac ttttgactaa atagctgaga ttcactttta tgtaaagtca ttttatagcg      540
   ttattaattt gggtgccttt aaaaatagta taaagcatgt ttctcgagtg tagtctgtta      600
   gccacctata ttggagagtt gggaggagag agtctctatc ttgaatttat gggaaaaatt      660
   ctaaaatact ttttataatg aaggacaaca tcataactcc ctaataaaat gtgcatgtat      720
   atattcaaat ttgctgtcat tgatcctgca cctacaaaat ccagtcctgg gggctggcat      780
   tcttactgct tgctgagggc cagatgatat agattccaga atatctccat gtagattttg      840
   gtgagaatta ctgtgctgaa aagaatgaca gtattgcagt tatacatggg ggttttggta      900
   ctttatattg tgactctgaa tttaaagcta tgcaatgtct tcttttttga aaggatataa      960
   ttgacactgg caaaacaatg cagactttgc tttccttggt caggcagtat aatccaaaga     1020
   tggtcaaggt cgcaaggtat gtatgacatt ttgacacaga atattttcct catttgaagg     1080
   gggattaagt gattgcttct ttttaaggat aaatgttttc aactgtcatt ttatcttcga     1140
   aaagtaatgt aatctcatat aagacttaag atataatcct tttaaataat tttgtcatgt     1200
   gttaataaag ctcataatta cagtcacttc cttgcctaat attaacattt ggtttttcag     1260
   catgctaatt atatcagttt gtcctgaata gcatggcaga ggattttggg cccccttgca     1320
   aaattaagaa taaggattcc aaagcgggtg aggaagtgat aggaaggggt gggccctgaa     1380
   gatctggacc tcctggaatt gagtgatgaa tgctgcatct tctttgtgtc tgtagtgaaa     1440
   ttttataatg cctgcttcct tttttattaa gtcggcctca cctcctcacc ttacctatgc     1500
   tgttttactt ttgcttttat agttctacct gtgtttattt ctcattttcg tttcatctct     1560
   caacaactct ggggtggcat tattattccc acttttcaga taaggttact gaggcatagg     1620
   gaattgtcca aaggtacaga gctagtccgc tatagagatg agatttgaac ccagggaacc     1680
   tggctcacag tttatgcttt tgctacctta agtttttaat agagtgacat caaacaaaca     1740
   tttaagaata tgtttttctt ttccttttat aatttcatta aaaacattaa gtctctgatc     1800
   agtctgcagt ttttatgtag gggtcaggta atgttctaac ttctgctttt tcctaagtga     1860
   ttaacaggtt tttataagcc cttttgaaaa aatcacggta tctgtcgagc atctttgaat     1920
   cagagtaagc cttctagtga gtcatatgtc agcagtttga ctgtatgggc ttttctaata     1980
   tccagttcaa gtgtttatca gtgagttttt cttttaaata gatttgggac aggtactatg     2040
   agagt                                                                  2045
<210> 27
   <211> 61
   <212> DNA
   <213> Artificial Sequence
<400> 27
   actagtctac cgttcgtata aagtatccta tacgaagtta ttagcttcat gtgagcaaaa 60
   g 61
<210> 28
   <211> 26
   <212> DNA
   <213> Artificial Sequence
<400> 28
   ggatcctcgc gtaggtggca cttttc 26
<210> 29
   <211> 61
   <212> DNA
   <213> Artificial Sequence
<400> 29
   ggatccataa cttcgtatag catacattat acgaagttat ccgccaagcc ctgtcccagg 60
   a 61
<210> 30
   <211> 72
   <212> DNA
   <213> Artificial Sequence
<400> 30
   actagtaagc ttacgcgtta ccgttcgtat aattatggtt atacgaagtt atacgatcat 60
   aatcagccat ac 72
<210> 31
   <211> 26
   <212> DNA
   <213> Artificial Sequence
<400> 31
   acgcgtcaac ctactgtggc atagac 26
<210> 32
   <211> 26
   <212> DNA
   <213> Artificial Sequence
<400> 32
   aagcttacag gcagaggtta tcctgg 26
<210> 33
   <211> 3001
   <212> DNA
   <213> Artificial Sequence
<400> 33
<210> 34
   <211> 26
   <212> DNA
   <213> Artificial Sequence
<400> 34
   aagcttagat cacagctcac tgtggc 26
<210> 35
   <211> 26
   <212> DNA
   <213> Artificial Sequence
<400> 35
   actagtcggc tgacacctgt aatctc 26
<210> 36
   <211> 2033
   <212> DNA
   <213> Artificial Sequence
<400> 36
   caacctactg tggcatagac tttaatcctc tgggtattct tttgttgttc tttcctggta 60
   tatgctgtgg aattgagata gactggttcg tgagcgagag attttgtgtt gccacaggta 120
   ggacatgctc aaacaatact tgggtcattt cttgacccaa gtcatctatt caccatagtt 180
   ttgtagcacc gatcttgcat acatttcatg tatcttcttt gaaccccacg tcagtgctgc 240
   ttatatgata ctcagaaatt aaacactaag gaataagatt ttcaggtagg attgagtttt 300
   ggagggtcac aaatcttgta atgtctaata tttccactct ccctgctgag aattagtttt 360
   ggcttccttg gaggtgatat cgcctctgtt gagtataagt ggcctactgt gatcacacca 420
   ctgcactcca gcctgggtga cagagtgaga ccctgtctca gaaaaaaaaa aaaaaaaaaa 480
   gaatgcatgg cctagatgac ttctaaggtt tttcccaccc agttccagtt ttcatgttct 540
   aggcagagca gtaaagtgag aaacacatgg acttgggagt ttagtctcgc atttcactgc 600
   cacttaatct gagcgactat tccatattta atctctctga atgtatttac tcatctttaa 660
   aggggaatga ttattaacat ctttttctca gggaaactat atgagtcaag gagataatat 720
   atttgaaaat ctttttaact gcaaagcgct gtttcactgt tggttataat gtgattgatc 780
   tcattgtagt gagcagctgc ttaattgcgt tttagaatgt agggaagata gtaatatttt 840
   tcacattata tatgtagctg gttctggaac tgtaaacata ctcctttttt atggagatct 900
   gagtcacgta ccataaaatt cactctttta aagttgtaca atccagtggt ttttgatata 960
   ttcagagttg tgcatctgct accactattt cattttggaa cccaaagaaa ccttgtaccc 1020
   attagcagtc attctccctt ctcccagccc ctggcaacta ctaatctact ttctacagaa 1080
   agtccgtaca gatttgtgta ttatggacat tccatataaa tggactcatg caatatcctg 1140
   tcttctttca cttagcatag tgttttcaag gttcatctag gttggggcat gtatcagtac 1200
   ttcatccctt gttttggctg aataatattt cattgtacaa atatatcaca ttttgcttat 1260
   ccatctgttg gtgaacattt gagtttctac ctgttggctt ttatgaataa tgttgatttg 1320
   aatgtttgtg tacaagtatg aatacctgtt ttcaggtctc ttgagtatat agttgctagg 1380
   tcatatagta actctgtgtt taacattttg aggaattgcc cgactattta acaaggtata 1440
   tgtactgttt tacaccagta acatatgagg gttccaatat ctccacatcc ttgacaacac 1500
   ttgttactgt cctttttatt gtagccatcc tagtggctat gatgtggtat ctcattgtgg 1560
   ttttgatttg tgtttctctg atgctgatga tgttgaacat gttttcatct gcttattggc 1620
   catttacata tatcttctta agaacggtta cccatttaca gtatggaaaa tgcttcagat 1680
   gcaactctag tcatgcctta gagatggagc tttattaaac attcagatct ctaggcatat 1740
   gaagtgctga gttctcttga actcctaata cagattgcac tgagtttagt gatacctttt 1800
   ctggagcatt cctgagttca ggtagggaga agggtttttg ctgtgattgg cttgttatgt 1860
   tctttctaaa tggaaataga attgaagtgt ctcctctctc catttattgg aagagtcatg 1920
   agggacataa ttagatgatc ccttggagtc tccggcttag gtcagtggtt atctacttag 1980
   gctgcacatt ggaatcacct gagagttaaa aaaccaggat aacctctgcc tgt 2033
<210> 37
   <211> 26
   <212> DNA
   <213> Artificial Sequence
<400> 37
   acgcgtagcc gaacctctcg caagtc 26
<210> 38
   <211> 30
   <212> DNA
   <213> Artificial Sequence
<400> 38
   tttggctttt aggggtagtt ttcacgacac 30
<210> 39
   <211> 58
   <212> DNA
   <213> Artificial Sequence
<400> 39
   atgataactt cgtatagcat acattatacg aagttatcca tggtgagcag ggcgagga 58
<210> 40
   <211> 60
   <212> DNA
   <213> Artificial Sequence
<400> 40
   tctagatacc gttcgtatag gatactttat acgaagttat taccacattt gtagaggttt 60
<210> 41
   <211> 55
   <212> DNA
   <213> Artificial Sequence
<400> 41
   ggtaccgaag ccgctagcgc taccggtcgc caccatgtcc aatttactgc cgtac 55
<210> 42
   <211> 26
   <212> DNA
   <213> Artificial Sequence
<400> 42
   agatctctaa tcgccatctt ccagca 26
<210> 43
   <211> 20
   <212> DNA
   <213> Artificial Sequence
<400> 43
   gacacatgca gacagaacag 20
<210> 44
   <211> 20
   <212> DNA
   <213> Artificial Sequence
<400> 44
   aacttctcgg ggactgtggg 20
<210> 45
   <211> 20
   <212> DNA
   <213> Artificial Sequence
<400> 45
   ctatcgcctt cttgacgagt 20
<210> 46
   <211> 20
   <212> DNA
   <213> Artificial Sequence
<400> 46
   gtttgctaac accccttctc 20
<210> 47
   <211> 19
   <212> DNA
   <213> Artificial Sequence
<400> 47
   gggagctcaa aatggagga 19
<210> 48
   <211> 20
   <212> DNA
   <213> Artificial Sequence
<400> 48
   gaagagttct tgcagctcgg 20
<210> 49
   <211> 20
   <212> DNA
   <213> Artificial Sequence
<400> 49
   gcttgcattg tatgtctggc 20
<210> 50
   <211> 20
   <212> DNA
   <213> Artificial Sequence
<400> 50
   ggttctttcc gcctcagaag 20
<210> 51
   <211> 20
   <212> DNA
   <213> Artificial Sequence
<400> 51
   gggagctcaa aatggaggac 20
<210> 52
   <211> 20
   <212> DNA
   <213> Artificial Sequence
<400> 52
   gcaataggtc aggctctcgc 20
<210> 53
   <211> 20
   <212> DNA
   <213> Artificial Sequence
<400> 53
   gaatgggttg gcggagctac 20
<210> 54
   <211> 21
   <212> DNA
   <213> Artificial Sequence
<400> 54
   gctgtggttc tgattgtgtg g 21
<210> 55
   <211> 20
   <212> DNA
   <213> Artificial Sequence
<400> 55
   gggagctcaa aatggaggac 20
<210> 56
   <211> 21
   <212> DNA
   <213> Artificial Sequence
<400> 56
   gctgtggttc tgattgtgtg g 21
<210> 57
   <211> 20
   <212> DNA
   <213> Artificial Sequence
<400> 57
   gactacaagt gcgtgccatc 20
<210> 58
   <211> 21
   <212> DNA
   <213> Artificial Sequence
<400> 58
   gctgtggttc tgattgtgtg g 21
<210> 59
   <211> 23
   <212> DNA
   <213> Artificial Sequence
<400> 59
   acgaggaccc tgaagtgaag ttc 23
<210> 60
   <211> 24
   <212> DNA
   <213> Artificial Sequence
<400> 60
   tgttcctctc taggcttggt cttg 24
<210> 61
   <211> 17
   <212> DNA
   <213> Artificial Sequence
<400> 61
   tacgtggacg gcgtgga 17
<210> 62
   <211> 22
   <212> DNA
   <213> Artificial Sequence
<400> 62
   tctactccgc ctccttcctg ta 22
<210> 63
   <211> 22
   <212> DNA
   <213> Artificial Sequence
<400> 63
   tggcagtagt aggtggcgaa gt 22
<210> 64
   <211> 15
   <212> DNA
   <213> Artificial Sequence
<400> 64
   atccggcacc gactt 15
<210> 65
   <211> 3097
   <212> DNA
   <213> Artificial Sequence
<400> 65
   aagcttgctc attgacactc tgtacccatt aatcctcctt attagctccc cttcaatgga      60
   cacatgggta gtcagggtgc aggtctcaga actgtccttc aggttccagg tgatcaacca     120
   agtgccttgt ctgtagtgtc aactcattgc tgccccttcc tagtaatccc cataatttag     180
   ctctccattt catagtcttt ccttgggtgt gttaaaagtg accatggtac actcagcacg     240
   gatgaaatga aacagtgttt agaaacgtca gtcttctctt ttgtaatgcc ctgtagtctc     300
   tctgtatgtt atatgtcaca ttttgtaatt aacagcttgc tggtgaaaag gaccccacga     360
   agtgttggat ataagccaga ctgtaagtga attacttttt ttgtcaatca tttaaccatc     420
   tttaacctaa aagagtttta tgtgaaatgg cttataattg cttagagaat atttgtagag     480
   aggcacattt gccagtatta gatttaaaag tgatgttttc tttatctaaa tgatgaatta     540
   tgattctttt tagttgttgg atttgaaatt ccagacaagt ttgttgtagg atatgccctt     600
   gactataatg aatacttcag ggatttgaat gtaagtaatt gcttcttttt ctcactcatt     660
   tttcaaaaca cgcataaaaa tttaggaaag agaattgttt tctccttcca gcacctcata     720
   atttgaacag actgatggtt cccattagtc acataaagct gtagtctagt acagacgtcc     780
   ttagaactgg aacctggcca ggctagggtg acacttcttg ttggctgaaa tagttgaaca     840
   gctttaatat acaataattg ttgcattatt atttcagatg ataaatgtgg tcataagtaa     900
   gaaataaatg atcgagttta gtcttttaat tcactgtcct ttgaatacct gcctcttact     960
   ctggaggcag aagtcccatg gatgtgttta tgaacatggt tgaggaagat ttaggaagac    1020
   tgcaacagta cactacctaa agtcgcgatg aaagtgtgag ttccggagcc tgacaactga    1080
   ttcaaagcat ggcttagtac ttcctaactc tgaccttggg caagttactt aacctctctg    1140
   tgtcccatat gtgattaggg tgaggttgat aatagcagcc atagagttaa gaggattaag    1200
   tgctataatg caagtagagc tcttacaaca gtttctggta aatcactcaa taaattcaga    1260
   catactatta ttttaagaaa tctcaaagag ttttcttgta ccttaaaatt ctcctagtgt    1320
   gaaccattgg ttttggtata ttgtgcttcc atgtagttta atatcaagat gtttttagat    1380
   ttccctttta atttatttgt tgacccattg gttgttcagg agcatgctgt ttacctgaaa 1440
   ataatggaga tattaaggta tttgaatatt tatcttctag tacattgaaa aactttttga 1500
   gagtaaccaa taataaatga tggaatgcta ctgctttttt tttttgaagc tgccagttat 1560
   tgtttactta cactatgcca aatataaagg cattaatctc ataaaagttt cacaacaatc 1620
   ctgtgaggga gacgatatcc ccattttaca aatcaggaaa ttaagactta ataaggttaa 1680
   aagacttgcc caaagtcaca gaaccagtaa gtggtagagc ttgaatttga atacagacct 1740
   gactctaaag ctcttttctt tctttagatt ttagtgttca ttgcttactt gaatgagtat 1800
   ctataagaaa actttaacat gtaaaacttc tgtgaaatta tcttgtccca tatcagggtc 1860
   atgtcaaact aatgtcctcc tcagcatctt tggaaaactt cagaggagaa atgagctttg 1920
   cccctcctgt tcatttcata taccactgtt agacctgtcc ttccctttca gcatgctttg 1980
   tccatattta gaagctgttg aagccattac ttgtctggtc agtttttagt gctggaatgg 2040
   acctagcctt ttaggccttc tgagatttag tttgatctcg tctttcccac ctaatggctc 2100
   tgttctacta catagatttg atctgaaaca gttctctgtt tctaaaataa ctttcttttc 2160
   atgatagtca cagtaaagta catttattat ggaaaaatca ataagtataa cgagtgaaag 2220
   ttatttcttg gtggtaagat tatgggatta tttgaacttt ctgtttcatt gtattttatt 2280
   tatttattta tttttgtgat ggagtctcac tctgctgccc aggctggagt gcagtagtac 2340
   gatcttggct cactgcaacc tccccttccc agttcaagtg attctcctgc ctcagactcc 2400
   caagtagctg ggattacagg cgcacgccac catgcctggc taattttttt atctttagta 2460
   gagacagggt ttcaccatgt tgaccaggct gatctccaac tcctgatctc aggtatccac 2520
   ctgcctcagc ctcccaaagt accgggatta cgggtgtgag ccaccctgcc tggcctcatt 2580
   ttgtcttttg ggggtatttt tgtgtgcaga tatatatgta tataaatatt tttccctctt 2640
   ttccccagtt agtatttgag cagatgaact ttggacccga atacctgtat tcaagtctct 2700
   aataccactt cttggctatt ttcattttat caaatggcct cttatcctcg tttttctcat 2760
   ttattaagta gagatgtaac tacttgatat aattcaaaaa ctcaataatg gcattctttt 2820
   gttttttaga ctctagtgtc tgtactcgtt gtaccatgct gggattcatt tgaacaattg 2880
   catggctttt ttagtgtatt attaaatttg cagtttactt agaatttact gggacctcat 2940
   acaaatggga aaaaaacata actgtgttac tcatttgctg tgtgcctttg gattgaccct 3000
   attttttgta ttcattttct ccccatgtcc tgagttccac tttgaataaa aagtaatttt 3060
   tttcctgcct gtaaaatagg ctaccaatag gactagt 3097
<210> 66
   <211> 20
   <212> DNA
   <213> Artificial Sequence
<400> 66
   tcccaaagta ccgggattac 20
<210> 67
   <211> 541
   <212> DNA
   <213> Artificial Sequence
<400> 67
   aatctttgtc agcagttccc ttttaaatgc aaatcaataa attcccaaaa atttaactgc 60
   tttatgaatt caatttaaaa atccttaaaa taagtcctgt ctctttaaaa aaacctatgc 120
   atagttatca tttctctaca aattaaccta gtttagtttt ctgttggttc cattttcctt 180
   gtttgttaag ttttagtagc tagtttaatt gtaatctcaa tgattatgtg gtagaatggg 240
   ttggcggagc tacaaaaatt cctagctact tcagagacat taaatttcag acacatggta 300
   cactttatat tacattttac tatgctaaaa taacacggct ttcttttgga attctgttca 360
   gtttttcaga ttgtaatctc agctacatct caacagattg ttctcagata tgtcctatta 420
   ccttctttgt gtagatagtg ctttattgac taagaacaat gacaacaaca ccttttgttt 480
   tctgggaata ggagaaaagt tttaagccaa aactcttaat tgcttatctg ctccacgtga 540
   g 541
<210> 68
   <211> 26
   <212> DNA
   <213> Artificial Sequence
<400> 68
   aagcttaatc tttgtcagca gttccc 26
<210> 69
   <211> 493
   <212> DNA
   <213> Artificial Sequence
<400> 69 gtactatgag agt 493
<210> 70
   <211> 502
   <212> DNA
   <213> Artificial Sequence
<400> 70
   gggggtattt ttgtgtgcag atatatatgt atataaatat ttttccctct tttccccagt 60
   tagtatttga gcagatgaac tttggacccg aatacctgta ttcaagtctc taataccact 120
   tcttggctat tttcatttta tcaaatggcc tcttatcctc gtttttctca tttattaagt 180
   agagatgtaa ctacttgata taattcaaaa actcaataat ggcattcttt tgttttttag 240
   actctagtgt ctgtactcgt tgtaccatgc tgggattcat ttgaacaatt gcatggcttt 300
   tttagtgtat tattaaattt gcagtttact tagaatttac tgggacctca tacaaatggg 360
   aaaaaaacat aactgtgtta ctcatttgct gtgtgccttt ggattgaccc tattttttgt 420
   attcattttc tccccatgtc ctgagttcca ctttgaataa aaagtaattt ttttcctgcc 480
   tgtaaaatag gctaccaata gg 502
<210> 71
   <211> 26
   <212> DNA
   <213> Artificial Sequence
<400> 71
   aagcttgggg gtatttttgt gtgcag 26